# EUROPEAN PATENT APPLICATION

(11) **EP 1 639 941 A1**
(43) Date of publication of application: **29.03.2006**
(21) Application number: 04746400.3
(22) Date of filing: 18.06.2004
(51) Int. Cl.: A61B 5/11, A61B 5/00, A61G 7/05, A47C 27/00

(54) **SLEEPING DEVICE AND SLEEPER'S IN-BED STATE DETECTION METHOD**

(30) Priority: 20.06.2003 JP 2003177087; 23.06.2003 JP 2003178103; 08.07.2003 JP 2003193430
(71) Applicant: MATSUSHITA ELECTRIC INDUSTRIAL CO., LTD., Kadoma-shi, Osaka 571-8501 (JP)
(72) Inventor: UEDA, Shigeki, Nara 630-1055 (JP); OGINO, Hiroyuki, Nara-shi Nara 630-8024 (JP)
(74) Representative: Grünecker, Kinkeldey, Stockmair & Schwanhäusser Anwaltssozietät
(86) International application number: PCT/JP2004/008931
(87) International publication number: WO 2004/112611

(57) **Abstract**

An object of the invention is to provide a bed which can surely detect the positional shift and heartbeat vibration of a bedded person while suppressing the increase of the cost and the degradation of the comfortability of a bed, and to provide a bed and an in-bed state detection method which can prevent the undesirable caught at the time of elevating the bed.

In order to detect the shift of a bedded person, a ratio of intensities between output signals from first pressure sensitive sensor 21 and a second pressure sensitive sensor 22 each having a cable shape is obtained. These sensors are disposed along the lying direction at the both end sides of a bedding surface 15a on which a bedded person lies, respectively. When the ratio is in a range of a predetermined shift state set in advance, it is determined that the positional shift of a bedded person on the bedding surface 15a occurs.

Further, a low-repulsion urethane layer is provided within a bed pad thereby to detect the heartbeat vibration. Furthermore, the elevational driving of the panel portion is controlled in accordance with the output form the pressure sensitive sensor disposed on a bed.

## Description

### <Technical Field>

The present invention relates a bed and an in-bed state detection method for monitoring the in-bed state of a person in the bed.

### <Background Art>

Recently, as a detection device for a bedded person in a bed, JP-A-7-88089, for example, discloses a device in which a pressure sensitive sensor for detecting whether a cared person as a bedded person is in bed or not and for detecting the change of the physical condition of the cared person is provided at a bed disposed in a hospital or various kinds of care facilities etc. This bed is arranged in a manner that many sensors each generating a small voltage due to the piezoelectric effect are disposed in the width direction of the bed on the upper surface of the bed on which the bedded person lies, and a row of the sensors thus disposed are arranged with a predetermined interval in the longitudinal direction of the bed. The voltages from these sensors are detected thereby to confirm whether a cared person is in bed or not and manage the change of the physical condition of the cared person.

JP-A-10-229973 discloses a biomedical monitor apparatus in which piezoelectric sensors each having a long tape shape are disposed on the upper surface of a bed in the width direction of the bed thereby determine a blood pressure value and an arteriosclerosis intensity of a bedded person by using a vibration detection means formed by these sensors.

However, like the first example, in the bed in which the sensors formed by the many piezoelectric elements are provided on the upper surface of the bed on which the bedded person lies, many expensive sensors are disposed in a lattice pattern, so that the cost of the bed is inevitably raised. Further, since the sensitivity of the sensor itself is relatively low, the sensors are required to be provided almost just on the upper surface of the bed on which the bedded person lies, whereby the comfortability of the bed is degraded.

Further, like the second example, also in the bed in which the piezoelectric sensors each having the long tape shape are disposed in the width direction, it is possible to detect whether a person is in bed or not, but it is impossible to monitor the positional shift of the person on the bed to prevent the person from falling etc. in advance. Further, also in the case of this bed, since the piezoelectric sensors are provided on the upper surface of the bed on which the bedded person lies, it is inevitable to degrade the comfortability of the bed.

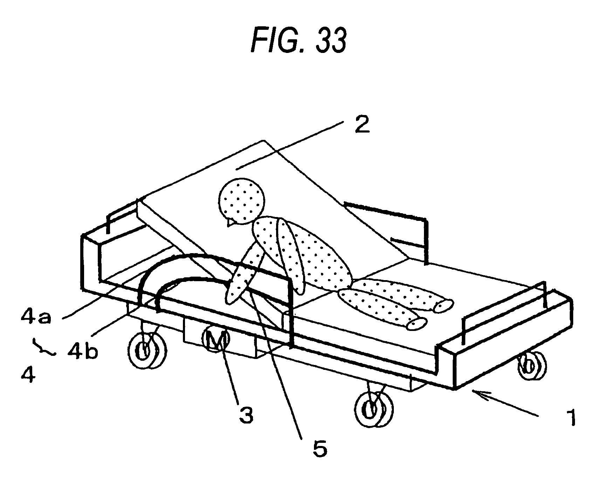

As another bed, JP-A-2003-52765 discloses an electrically operated bed in which the panel portion of the bed is adjustable and which is provided with a side fence also serving as an assistant means for prevent a quilt or a patient from falling. The conventional electrically operated bed of this type will be explained with reference to Fig. 33. In the figure, 1 depicts the electrically operated bed, 2 a back raising panel portion, 3 a panel portion adjusting means and 4 a fence portion formed by pipe members. The fence portion 4 is configured by an outer frame pipe 4a and an inner frame pipe 4b which are bent in an arc shape so as to be in parallel to each other and which arc shape is made coincide with a locus along which the back raising panel portion 2 moves elevationally. Thus, even if the back raising panel portion 2 is raised while a hand 5 is inserted between the outer frame pipe 4a and the inner frame pipe 4b, the hand 5 slidably moves up on the outer frame pipe 4a or the inner frame pipe 4b along the elevational locus of the back raising panel portion 2, so that the hand is prevented from being caught.

However, the conventional electrically operated bed of this type has a problem that when the back raising panel portion 2 is raised while a part of a human body is inserted between the outer frame pipe 4a and the inner frame pipe 4b, the part of the human body becomes hard to slide on the outer frame pipe 4a or the inner frame pipe 4b due to a posture or a physique and a friction characteristics etc. of the human body or the cloth surface, so that the part of the human body is caught inadvertently.

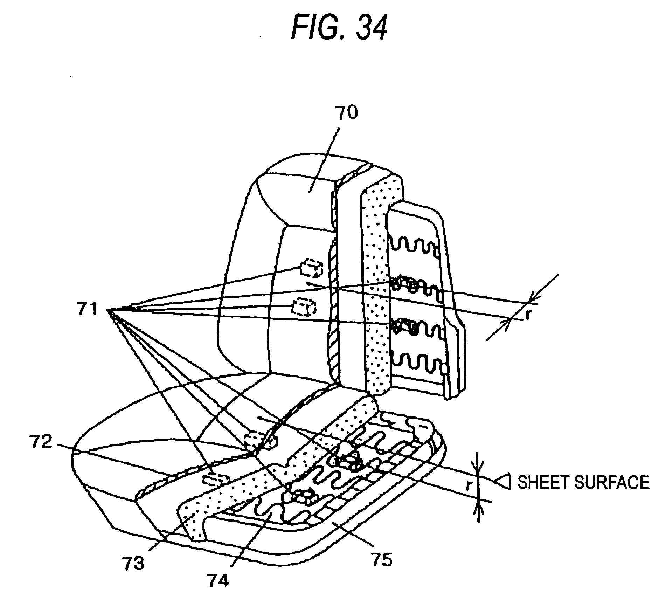

As another bed, JP-A-8-282358 discloses an apparatus provided with a pad which can detect the heartbeats and the movement of breathing of a human body by using a pressure sensitive sensor. Fig. 34 is a diagram showing the configuration of the conventional human body detecting apparatus.

In the figure, 70 depicts a seat, 71 a vibration detecting means, 72 a surface cloth, 73 an urethane foam, 74 a seat spring and 75 a seat frame. Each of the plural vibration detecting means 71 is fixed on the seat spring 74 constituting the seat 70 which is away by a constant distance r or more from the contact surface of the seat 70 with a human body via the surface cloth 72 and the urethane foam 73. In this case, r is the distance at which the presence of the vibration detecting means 71 is not felt by a human body seated on the seat 70. This distance becomes longer as the hardness of the outer casing of the vibration detecting means 71 is higher and the urethane foam 73 becomes softer.

In this manner, each of the plural vibration detecting means is fixed at the portion away from the surface of the seat made of urethane by the constant distance or more and the outputs of the plural vibration detecting means are processed to determine whether a human body exists on the seat or not. Since the plural vibration detecting means are disposed away from the contact surface with the human body, influence on the seating feeling can be reduced even when the plural vibration detecting means are configured by rigid parts.

However, this invention relates to a seat for a car. In the case of the seat for a car, since the entire weight of the upper part of a body higher than the hips is concentrated on the narrow hips, the detection can be made even if the vibration detecting means is provided at the lower surface of the seat made of urethane. When this technical concept was applied to a bed as it is, it was found that there arose the following problem.

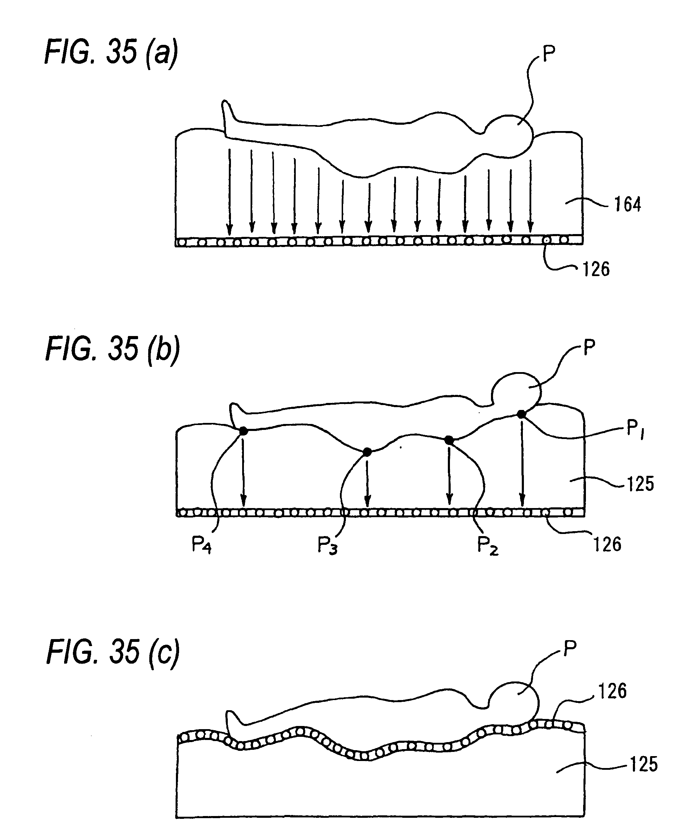

Figs. 35(a) to 35(c) show various kinds of the experimental examples in each of which the plural vibration detecting means (piezoelectric sensors) are used for a bed. Fig. 35(a) shows the experimental example 1, Fig. 35(b) shows the experimental example 2 and Fig. 35(c) shows the experimental example 3. In Fig. 35(b), 125 depicts a bed formed by an urethane layer and 126 a piezoelectric sensor disposed beneath the urethane layer bed 125. In this case, particularly, the piezoelectric sensor of a cord shape (described later) is used. P depicts a human body lying on the bed.

The signals (heartbeats generated from the heart of a human body) obtained from the cord shaped piezoelectric sensors 126 were detected in a state where a person lay down on the urethane layer bed 125 having the cord shaped piezoelectric sensors 126 disposed at the lower portion thereof, whereby the detection values detected by the cord shaped piezoelectric sensors 126 were small values.

The applicant of this invention considered that the reason of this results was as follows. That is, when a person lay on the urethane layer bed, the protruding portions on the back side of the human body P, that is, a head portion P1, shoulder blade portions P2, hip portions P3 and heel portions P4 abutted against the bed pad with strong forces and so sank deeply. However, since other portions of the human body did not sink so much, only these four portions served as vibration sources and so the heartbeat vibration from these portions was transmitted downward. As a result, a sufficient signal could not be detected even from the cord shaped piezoelectric sensors 126.

When the amplitude was increased, other noises (vibrations of cars passing outside, vibrations of persons passing near the bed, etc.) were caught much and so it was proved that the apparatus might be practical for car seats but not practical for the bed of Fig. 35(b).

Fig. 35(c) shows an experimental example for covering the aforesaid drawback of Fig. 35(b) thought by the applicant of this invention, in which the cord shaped piezoelectric sensors 126 were disposed on the urethane layer bed 125. When a person lay on such the bed, the sensors could surely detect heartbeat vibration not only from the head portion P1, the shoulder blade portions P2, the hip portions P3 and the heel portions P4 but also from other portions such as a neck portion, a waist portion, thigh portions, calf portions.

However, since a person lay on the sensor cable with a diameter of several millimeters which was bent for many times and disposed on the bed, there was a practical problem that the comfortability of the bed is not good.

The first invention of this application has been made in view of the aforesaid circumstances and an object of the first invention is to provide a bed and an in-bed state detection method which can surely detect the positional shift of a bedded person thereby to be able to prevent the fall of the person in advance while suppressing the increase of the cost and the degradation of the comfortability of a bed.

Further, an object of the second invention of this application is to provide a bed which can prevent the undesirable caught at the time of elevating a bed.

Furthermore, an object of the third invention of this application is to provide a bed having a bed pad which can surely detect heartbeat vibration and is comfortable to sleep.

### <Disclosure of the invention>

The aforesaid objects can be attained by the following configurations.
(1) A bed for detecting shift of a position of a bedded person on a bedding surface, comprising:
   a first pressure sensitive sensor and a second pressure sensitive sensor each having a cable shape, disposed separately along side edges of the bedding surface at both end sides in a width direction of the bedding surface, respectively, each of which detects an acceleration component caused by a movement of a bedded person; and
   determination means which compares output signals from the first pressure sensitive sensor and the second pressure sensitive sensor to determine a shift state of the bedded person toward a side potion of the bedding surface.

   According to this bed, the first detection sensor and the second detection sensor each formed by the cable-shaped pressure sensitive sensor are disposed at positions along the side edges of the bedding surface at the both end sides of the bedding surface, then the determination means compares the output signals from the first detection sensor and the second detection sensor thereby to determine the positional shift of a bedded person on the bedding surface toward the side portion, thereby preventing the bedded person from falling from the bedding surface in advance in accordance with the determination result. Further, since the cable-shaped pressure sensitive sensors are merely disposed along the both end sides of the bedding surface, the cost-up can be suppressed as much as possible and also the degradation of the comfortability to sleep can be suppressed as much as possible as compared with a case where sensors are disposed in a lattice pattern.
(2) The bed according to (1) is characterized in that the first pressure sensitive sensor and the second pressure sensitive sensor are arranged in a manner that a wiring density on a head portion side of the bedded person is set to be higher than other region.
   According to this bed, since the wiring density of the pressure sensitive sensors on the head portion side of a bedded person is set to be higher, the detection sensitivity of a head portion is particularly improved. Thus, even in a case when such a shift arises that a bedded person lays his/her head near the side end portion on the bedding surface, the head portion of the bedded person can be detected with a high sensitivity thereby to accurately and surely grasp the shift state of the bedded person.
(3) The bed according to (1) or (2) is characterized in that the first pressure sensitive sensor and the second pressure sensitive sensor are arranged in a manner that a wiring density on a leg portion side of the bedded person is set to be lower than other region.
   According to this bed, since the wiring density of the pressure sensitive sensor on the leg portion side of the bedded person is set to be lower, the detection sensitivity on the leg portion side becomes low. Thus, even when a shift state does not occur such that a bedded person lies at the center portion on the bedding surface with the person's leg being opened, it is possible to eliminate such a problem of an erroneous determination that the position of the bedded person is shifted.
(4) The bed according to (2) or (3) is characterized in that the first pressure sensitive sensor and the second pressure sensitive sensor are wired in a wave shape on the bedding surface, and the wiring density is changed in accordance with an interval between adjacent waves of the wave shape.
   According to this bed, since each of the pressure sensitive sensors is wired in the wave shape on the bedding surface, and the wiring density is changed in accordance with the interval between adjacent waves of the wave shape, the wiring density can be arbitrarily set with ease.
(5) The bed according to (1) to (4) is characterized in that projection pieces are disposed on an upper side or a lower side of the pressure sensitive sensor and are overlapped on the pressure sensitive sensor at plural portions thereof.
   According to this bed, since the projection pieces are disposed on the upper side or the lower side of the pressure sensitive sensor so as to be overlapped on the pressure sensitive sensor at plural portions thereof, the weight or movement of a bedded person can be acted on the pressure sensitive sensors as a large force locally, so that a large signal can be outputted and the detection sensitivity of the positional shift of a bedded person can be enhanced.
(6) The bed according to (5) is characterized in that the pressure sensitive sensor is adhered to a sheet and the projection pieces are adhered to a sheet, and the pressure sensitive sheet to which the pressure sensitive sensor is adhered and the projection piece sheet to which the projection pieces are adhered are laminated to each other.
   According to this bed, the sensitivity of the pressure sensitive sensor can be improved with a quite simple configuration by merely laminating to each other the pressure sensitive sheet at which the pressure sensitive sensor is provided and the projection piece sheet at which the projection pieces are provided.
(7) The bed according to (1) to (6) is characterized in that the determination means outputs a notification signal when it is determined that positional shift of the bedded person on the bedding surface arises, and notification means is further provided which notifies occurrence of shift in response to the notification signal outputted from the determination means.
   According to this bed, since the notification means notifies in response to the notification signal outputted from the determination means, the positional shift of a bedded person can be notified to a third party such as a cared person, so that the shift can be corrected quickly.
(8) An in-bed detection method of a bed for detecting shift of a position of a bedded person on a bedding surface, characterized by comprising the steps of:
   obtaining a ratio between intensities of output signals from a first pressure sensitive sensor and a second pressure sensitive sensor each having a cable shape, disposed separately along side edges of the bedding surface at both end sides in a width direction of the bedding surface, respectively, each of which detects an acceleration component caused by a movement of a bedded person; and
   determining that positional shift of the bedded person on the bedding surface arises when the ratio is in a predetermined range set in advance.

   According to this bed, a ratio between intensities of the output signals from the first pressure sensitive sensor and the second pressure sensitive sensor is obtained, and it is determined that positional shift of the bedded person on the bedding surface arises when the ratio is out of the predetermined range set in advance. Thus, the positional shift of a bedded person can be surely and accurately detected in spite of the simple configuration.
(9) In a bed which includes at least one of a back raising panel portion and a knee raising panel portion and driving means which performs elevational driving of at least one of the back raising panel portion and the knee raising panel portion, and which is capable of attaching a side fence, the bed comprising:
   a pressure sensitive sensor which is disposed at an entire periphery or a part of a surface end portion of at least one of the back raising panel portion and the knee raising panel portion;
   determination means which determines touch of a bedded person to the pressure sensitive sensor based on an output signal from the pressure sensitive sensor; and
   control means which controls the driving means in accordance with a determination signal from the determination means.

   According to this bed, when it is determined that a bedded person contacts with the pressure sensitive sensor during the elevational operation of the back raising panel portion or the knee raising panel portion, such a control can be performed that the elevational driving of the back raising panel portion or the knee raising panel portion is stopped or the driving direction is reversed. As a result, an inadvertent caught at the time of the elevational movement of a bed can be prevented.
(10) The bed according to (9) is arranged in that the pressure sensitive sensor is formed by a non-linear flexible member which displacement amount with respect to a load is non-linear and a piezoelectric sensor with flexibility which deforms according to a displacement of the non-linear flexible member.
   According to this bed, when the pressing load of a bedded person against the pressure sensitive sensor is the predetermined value or more, the non-linear flexible member deforms abruptly. Thus, since the piezoelectric sensor also deforms abruptly due to such a displacement of the non-linear flexible member, the output signal having a magnitude sufficient for detecting the contact due to the movement of the bedded person can be obtained from the pressure sensitive sensor.
(11) The bed according to (10) is aranged in that the non-linear flexible member is configured by a thin type elastic material which is formed in a belt shape and has a convex portion.
   According to this bed, since the non-linear flexible member described in (10) or (11) and the piezoelectric sensor are disposed in the deformation means capable of deforming according to a load, the non-linear flexible member and the piezoelectric sensor are further likely deformed when a load is applied, so that the sensitivity of the pressure sensitive sensor can be further improved.
(12) The bed according to (10) or (11) is arranged in that the non-linear flexible member and the piezoelectric sensor are disposed in deformation means capable of deforming according to a load
   According to this bed, the non-linear flexible member and the piezoelectric sensor are further likely deformed when a load is applied, so that the sensitivity of the pressure sensitive sensor can be further improved.
(13) The bed is arranged in that the deformation means described in (4), in particular, has a hollow portion so that at least one of the non-linear flexible member and the piezoelectric sensor can be deformed easily.
   According to this bed, the practical configuration of the deformation means can be realized.
(14) The bed is arranged in that the determination means in one of (1) to (5), in particular, determines whether or not a bedded person continues to touch the pressure sensitive sensor based on an output signal from the pressure sensitive sensor.
   According to this bed, the piezoelectric type pressure sensitive sensor can realize the operation similar to that of a pressure sensitive sensor of a static load detection type, the usability can be improved.
(15) The bed is arranged in that the control means in one of (9) to (14) is arranged to validate the determination signal from the determination means when the driving means performs the elevational driving and to invalidate the determination signal from the determination means when the driving means does not perform the elevational driving.
   According to this bed, even if a bedded person or a third party touches the pressure sensitive sensor during the stationary state of the back raising panel portion, for example, the back raising panel portion is prevented from being inadvertently driven elevationally. Since the determination signal as to the contact to the pressure sensitive sensor is made valid only when the back raising panel portion is driven elevationally, it is possible to provide the electrically driven bed which provides the sense of security and does not perform erroneous operation.
(16) The bed is arranged by comprising:
   a low-repulsion urethane layer; and
   a pressure sensitive sensor disposed on a lower surface of the low-repulsion urethane layer.

   According to this bed, the heartbeat vibration etc. can be surely detected and a bed pad being comfortable to sleep can be obtained.
(17) The bed is arranged by comprising:
   a low-repulsion urethane layer;
   a cushion lawyer made of usual urethane disposed on a lower surface of the low-repulsion urethane layer; and
   a pressure sensitive sensor disposed on a lower surface of the cushion lawyer.

   According to this bed,
   According to this bed, the heartbeat vibration etc. can be surely detected and a bed pad being comfortable to sleep can be obtained with a low cost.
(18) The bed according to (16) or (17) is arranged in that the pressure sensitive sensor is configured by a cord-shaped piezoelectric sensor with flexibility.
   According to this bed, the heartbeat vibration etc. can be further surely detected.
(19) The bed according to one of (16) to (18) comprising:
   a rigid plate having unevenness formed on a surface thereof; and
   a bed pad which is disposed on the rigid plate and recited in one of claims 16 to 18.

   According to this bed, the heartbeat vibration etc. can be further surely detected.
(20) The bed is arranged by comprising:
   a bed frame;
   a movable plate attached on the bed frame so as to be able to incline; and
   a bed element which is disposed on the movable plate and described in (19).

According to this bed, the heartbeat vibration can be surely detected and a care bed being comfortable to sleep can be obtained.

### <Brief Description of the Drawings>

Fig. 1 is an exploded perspective view of the bed according to the first embodiment of the invention.
Fig. 2 is a plan view of a pressure sensitive sheet constituting the detection device of the bed.
Fig. 3 is a plan view showing a pressure sensitive sensor and projection pieces of a projection sheet.
Fig. 4 is a sectional view showing the pressure sensitive sensor and the projection sheet.
Fig. 5 is an exploded perspective view of the pressure sensitive sensor disposed on the pressure sensitive sheet.
Fig. 6 is a block diagram showing the constitution and the function of a detection device.
Fig. 7 is a graph for explaining the method of determining the positional shift using a determination means.
Figs. 8(a) to 8(c) are schematic plan views each showing the position of a bedded person on a bedding surface.
Fig. 9 is an exploded perspective view of a pressure sensitive sheet for explaining another constitution of the pressure sensitive sheet.
Fig. 10 is a schematic plan view of the pressure sensitive sheet constituting the detection device of the bed.
Fig. 11 is a schematic diagram of a bedded person which shows the distribution of a load of the bedded person to the bedding surface.
Figs. 12(a) to 12(c) are schematic plan views each showing the position of a bedded person on the bedding surface.
Fig. 13 is an external perspective view of the electrically driven bed according to the second embodiment of the invention.
Fig. 14 is an external perspective view of the apparatus in which pressure sensitive sensors are disposed at portions of the surface end portions of a back raising panel portion and a knee raising panel portion.
Fig. 15 is a schematic side view showing the positional relation between the bed and the side fence of the apparatus.
Fig. 16 is a schematic perspective view showing a state where a bedded person touches the pressure sensitive sensor.
Fig. 17 is an external view of the pressure sensitive sensor of the apparatus.
Fig. 18 is a schematic diagram of a main portion showing a state where the pressure sensitive sensor of the apparatus is fixed to the back raising panel portion and the knee raising panel portion by means of a sewing machine.
Fig. 19 is a perspective view of a main portion of the piezoelectric sensor serving as the pressure sensitive sensor of the apparatus.
Fig. 20 is an external view of a system of the piezoelectric sensor serving as the pressure sensitive sensor having a determination means of the apparatus.
Fig. 21 is an external view showing a state of the deformation when a load is applied to the pressure sensitive sensor of the apparatus.
Fig. 22 is a characteristic diagram showing a load W, a displacement L of the pressure sensitive sensor, an output signal D of the piezoelectric sensor and a determination output J of the determination means, when a load is applied to the pressure sensitive sensor of the apparatus.
Fig. 23 is a flowchart showing the determination procedure of the determination means of the apparatus.
Fig. 24(a) is an external perspective view in a case where the pressure sensitive sensor of the apparatus is disposed in a U-shape manner on the surface of the back raising panel portion at the upper end portion and the both end portions in the width direction of the back raising panel portion, and Fig. 24(b) is an external perspective view in a case where the pressure sensitive sensors of the apparatus are disposed on the surface of the back raising panel portion at the both end portions in the width direction of the back raising panel portion.
Fig. 25(a) is a sectional view showing a cushion state in a case where usual urethane foam is used in the apparatus, and Fig. 25(b) is a sectional view showing a cushion state in a case where low-repulsion urethane foam is used in the apparatus.
Fig. 26(a) is a sectional diagram of the configuration which is arranged to space between a piezoelectric sensor and a non-linear flexible member in the pressure sensitive sensor of the apparatus, Fig. 26(b) is a sectional diagram of the configuration which is arranged to be provided with a hollow portion in the pressure sensitive sensor of the apparatus, and Fig. 26(c) is a sectional diagram of the configuration which is arranged to dispose a sheet-shaped piezoelectric sensor in the pressure sensitive sensor of the apparatus.
Fig. 27(a) is an external perspective views of the configuration in which the pressure sensitive sensor is disposed on the rear surfaces of the back raising panel portion and the knee raising panel portion, Fig. 27(b) is an external perspective view of the configuration in which the pressure sensitive sensor is disposed on the side surfaces of the back raising panel portion and the knee raising panel portion, and Fig. 27(c) is an external perspective view of the configuration in which the pressure sensitive sensor is disposed in a zigzag manner on the side surfaces of a mattress.
Fig. 28 is an exploded perspective view of the bed according to the third embodiment of the invention.
Fig. 29(a) shows a case where a cord-shaped piezoelectric sensor is disposed on an uneven plate and Fig. 29(b) shows a case where the cord-shaped piezoelectric sensor is disposed on a flat plate.
Fig. 30 is a configuration diagram of the cord-shaped piezoelectric sensor.
Fig. 31 is diagrams showing a load applied to the cord-shaped piezoelectric sensor and sensor output characteristics thereof.
Fig. 32(a) shows a block diagram of a detection unit for detecting heartbeats and breathing and Fig. 32(b) shows a diagram for explaining the theory for converting into pulse signals the detection signal from the cord-shaped piezoelectric sensor generated in response to heartbeats, breathing or the movement of a body caused by turning-over etc.
Fig. 33 is an external perspective view showing a conventional electrically driven bed.
Fig. 34 is a constitutional diagram showing a conventional sheet.
Figs. 35(a) to 35(c) show various kinds of experimental examples using a piezoelectric sensor for a bed.

In the drawings, a reference numeral 11 depicts a projection sheet, 13, 55, 61 each depicts a pressure sensitive sheet, 15a depicts a bedding surface, 17, 53 each depicts a projection sheet, 21 depicts a pressure sensitive sensor (first pressure sensitive sensor), 22 depicts a pressure sensitive sensor (second pressure sensitive sensor), 23 depicts a center electrode, 25 depicts a piezo element material, 27 depicts an outer electrode, 29 depicts a sheath, 31 depicts a first signal processing unit, 33 depicts a second signal processing unit, 35 depicts an amplifier circuit, 37 depicts a filter, 39 depicts a smoothing circuit, 41 depicts a determination means, 43 depicts a notification means, 51, 52 each depicts a sheet, 50 depicts a bed, A, B each depicts an output, H depicts a bedded person, 80 depicts an electrically driven bed, 82 depicts a bedded person, 83 depicts a back raising panel portion, 84 depicts a knee raising panel portion, 85 depicts an elevationally driving unit, 86, 86a, 92, 93, 94, 110, 112, 114 each depicts a pressure sensitive sensor, 87 depicts a determination means, 88 depicts a side fence, 89 depicts a deformation means, 90 depicts a non-linear flexible member, 91 depicts a piezoelectric sensor (cable shaped), 97 depicts a flange portion, 98 depicts a center electrode, 99 depicts an outer electrode, 100 depicts a composite piezoelectric layer, 101 depicts a cover layer, 102 depicts a piezoelectric sensor end portion, 103 depicts a wire-breakage detection resistor, 104 depicts a cable, 105 depicts a connector, 107 depicts the side surface of a mattress, 108 depicts a low-repulsion urethane foam, 109 depicts a usual urethane foam, 111 depicts a hollow portion, 113 depicts a piezoelectric sensor (sheet shaped), 120 depicts a bed pad, 121 depicts a low-repulsion urethane layer, 122 depicts a usual urethane layer, 123 depicts a cord-shaped piezoelectric sensor laying sheet, 124 depicts a cloth sheet, 125 depicts a bed, 126 depicts a cord-shaped piezoelectric sensor, 127 depicts an uneven plastic plate, 128 depicts a plastic plate, 129 depicts a recess, 130 a cover bag, 131 depicts a sheet, 132 depicts a fastener, 134 depicts a bed frame, 135 depicts a picture-frame shaped frame, 136 depicts a movable plate, 137depicts a supporting plate, 138 depicts a motor, 145 depicts a detection unit for heartbeats and breathing etc., 146 depicts a cord-shaped pressure sensitive sensor (cord-shaped piezoelectric sensor), 147 depicts a filter circuit, 148 depicts an amplifier circuit, 149 depicts a counter circuit and 150 depicts a display unit.

### <Best Mode for Carrying Out the Invention>

Hereinafter, the preferred embodiments of a bed and an in-bed state detection method according to the invention will be explained with reference to drawings.

### Embodiment 1

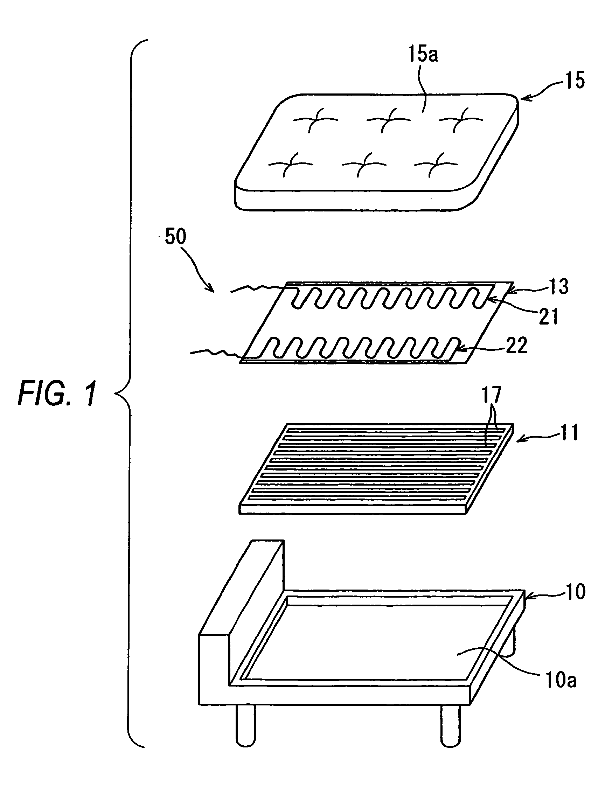

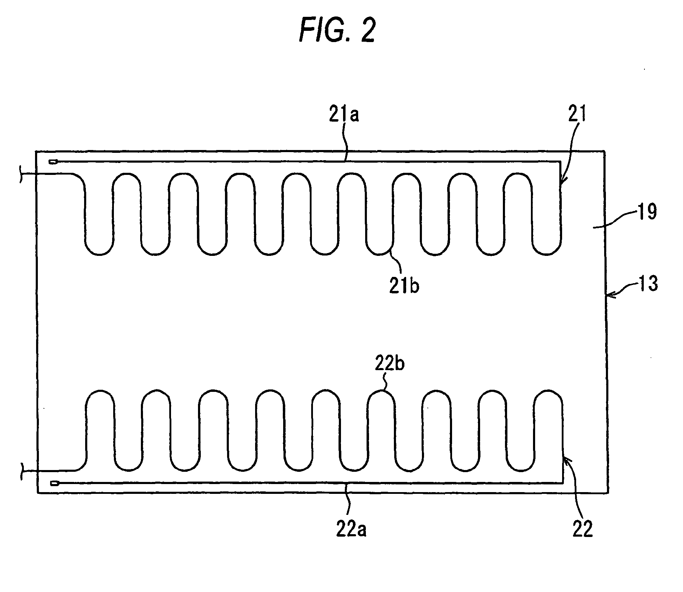

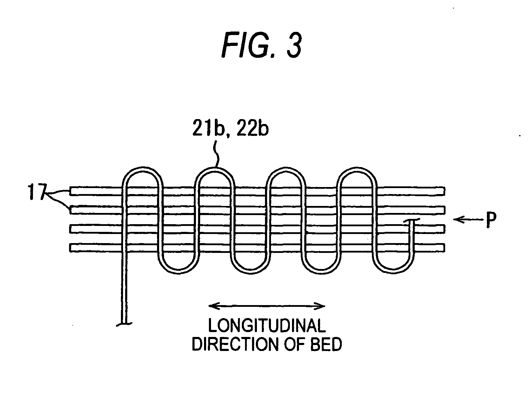

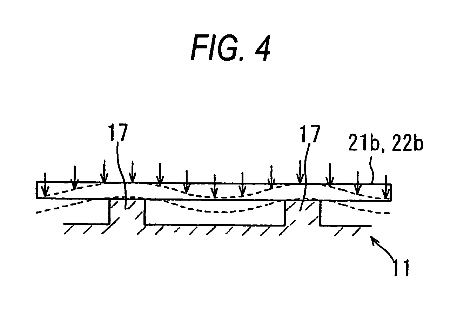

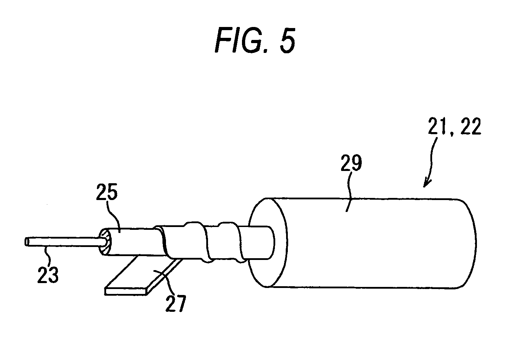

Fig. 1 is an exploded perspective view of the bed according to the embodiment 1, Fig. 2 is a plan view of a pressure sensitive sheet constituting the detection device of the apparatus, Fig. 3 is a plan view showing a pressure sensitive sensor and projection pieces of a projection sheet, Fig. 4 is a sectional view seen from a direction P in Fig. 3, and Fig. 5 is an exploded perspective view of the pressure sensitive sensor disposed on the pressure sensitive sheet.

As shown in Fig. 1, a bed (bed) 50 is configured in a manner that a projection sheet 11, a pressure sensitive sheet 13 and a mattress 15 are laminated on the panel surface 10a of a frame 10 in this order and assembled.

In the projection sheet 11, a plurality of projection pieces 17 are formed in parallel from one another along the longitudinal direction of the bed 50 on the upper surface side of the projection sheet. The projection pieces 17 are not limited to the configuration of the plural lines each formed by a linear continuous line shown in the figure, but may be projections of a linear shape provided intermittently, projections of a curved shape or projections provided discretely. The pressure sensitive sheet 13 constituting the detection device is laminated on the upper surface of the projection sheet 11 in a manner that pressure sensitive sensors are disposed on the lower side of the pressure sensitive sheet. The upper surface of the mattress 15 laminated on the upper surface of the pressure sensitive sheet 13 serves as a bedding surface 15a.

As shown in Fig. 2, the pressure sensitive sheet 13 has a sheet 19 of a rectangular shape in its plan view which is formed by a soft non-woven cloth, as an example, and a pair of the pressure sensitive sensors 21, 22 adhesively laminated at the both end sides of the sheet 19 respectively so as to have a space therebetween such that these sensors are sewn on the sheet 19.

The pressure sensitive sensors 21, 22, each of which is a piezoelectric sensor of a long cable shape using piezo element material, have straight line portions 21a, 22a disposed linearly along the edge portions of the both end sides of the sheet 19 and wave shaped portions 21b, 22b disposed in a wave-shaped fashion along the straight line portions at the inner sides of the sheet 19 than the straight line portions 21 a, 22a, respectively. In these pressure sensitive sensors 21, 22, as shown in Fig. 3, the wave shaped portions 21b, 22b cross in a plan view with the projection pieces 17 of the projection sheet 11 laid beneath the wave shaped portions.

Thus, the pressure sensitive sheet 13 provided with the pressure sensitive sensors 21, 22 is pushed down via the mattress 15 as shown in Fig. 4 due to the weight and the movement of the body of a bedded person laying on the bedding surface 15a of the mattress 15, so that parts of the wave shaped portions 21b, 22b of the pressure sensitive sensors 21, 22 are abutted against the projection pieces 17 of the projection sheet 11. In this manner, a load from the bedded person is locally applied against the pressure sensitive sensors 21, 22 crossing with the projection pieces 17. Thus, even if deformation amounts of the pressure sensitive sensors 21, 22 increase and so the deformation of the bedded person appears not only in the amplitude direction of the waved shape (width direction of the bed) but also in the direction orthogonal thereto(longitudinal direction of the bed), the deformation can be surely detected

Next, the concrete configuration of the pressure sensitive sensors 21, 22 will be explained.

As shown in Fig. 5, each of the pressure sensitive sensors 21, 22 is arranged in a manner that it has a center electrode 23 of a coil shape at the center portion along the axial direction thereof, piezo element material 25 is covered on the periphery of the center electrode 23, an outer electrode 27 is disposed on the outer periphery of the piezo element material, and the outermost periphery of the sensor is covered a sheath 29 made of vinyl chloride resin etc.

In each of the pressure sensitive sensors 21, 22, resin material having heat resistance durable to the usable temperature of 120 C° is used as the piezo element material 25. This piezo element material has characteristics of capable of being used in a temperature region (120 C° or less) higher than 90 C° which is the maximum usable temperature of the conventional typical high-molecular piezo element material (uniaxially oriented polyvinylidene fluoride) or piezo element material made from chloroprene and piezoelectric ceramic powder.

Further, the piezo element material 25 is formed by resin with flexibility and piezoelectric ceramics, the center electrode 23 is formed by coil-shaped metal, and a flexible electrode of a film shape is used as the outer electrode 27, whereby the sensor has flexibility similar to that of a normal vinyl cord.

Furthermore, each of the pressure sensitive sensors 21, 22 has a high sensitivity similar to that of high-molecular piezo element material and has a high sensitivity similar to that of high-molecular piezo element material in a low frequency range (10Hz or less) for detecting the motion of a human body. This is because the dielectric constant (about 55) of the piezo element material 25 is larger than that (about 10) of high-molecular piezo element material, so that the degree of the reduction of the sensitivity is low in even in the low frequency region (10 Hz of less).

The piezo element material 25 is constituted by complex, of resin material and piezoelectric ceramics powder of 10 µm or less. The vibration detection characteristics and the flexibility of the piezo element material are realized by ceramics and resin, respectively.

In the piezo element material 25, since chlorinated polyethylene is used as the resin material, flexibility facilitating the forming as well as high heat resistance (120 C°) are realized, and further simple manufacturing processes eliminating a bridging process can be realized.

Each of the pressure sensitive sensors 21, 22 thus obtained does not have piezoelectric efficiency so long as the piezo element material 25 is merely formed. Thus, it is necessary to perform a processing (polarization processing) for applying piezoelectric efficiency to the piezo element material 25 by applying a high DC voltage of several kV/mm to the piezo element material 25. This polarization processing is performed in a manner that after forming the center electrode 23 and the outer electrode 27 at the piezo element material 25, the high DC voltage is applied to the both electrodes 23, 27.

When a small defect such as a crack exists within the piezo element material 25, discharge likely occurs at the defect portion and so there likely arises the short-circuit between the both electrodes, so that a sufficient polarization voltage can not be applied. However, in the pressure sensitive sensors 21, 22, since a unique polarization process is established in which an auxiliary electrode capable of adhering to the piezo element material 25 of a constant length is used, a defect can be detected and avoided thereby to stabilize the polarization, whereby the sensor can be elongated so as to have a length of 10 m or more.

Further, in each of the pressure sensitive sensors 21, 22, since the coil-shaped metal center electrode is used as the center electrode 23 and the film-shaped electrode (three layer laminated film of aluminum, polyethylene terephthalate and aluminum) is used as the outer electrode 27, the adhesiveness between the piezo element material 25 and the respective electrodes 23, 27 is secured. Further, an external lead wire can be coupled easily and a flexible cable-shaped mount structure can be realized.

A copper-silver alloy coil is used as the center electrode 23, the three layer laminated film of aluminum, polyethylene terephthalate and aluminum is used as the outer electrode 27, polyethylene resin plus piezoelectric ceramic powder is used as the piezo element material 25 and thermoplastic plastics , whereby the dielectric constant becomes 55, an electric charge generation amount becomes 10 to 13 C (coulomb) / gf and the maximum usable temperature becomes 120 C°.

Each of the pressure sensitive sensors 21, 22 is arranged so as to output a signal only at a moment where a force is applied thereto and not to output any signal any more so longs as there is no deviation even if the force is continued to be applied thereto. Similarly, each of the sensors has characteristics of detecting acceleration such that a signal is outputted only at a moment where the force is removed. Thus, in each of the pressure sensitive sensors 21, 22, even when it is disposed in a bent manner, the sensor outputs a signal on a moment where it is bent but does not output a signal after the completion of the disposition. Thereafter, each of the pressure sensitive sensors 21, 22 outputs a signal only when a force is applied to a portion thereof.

Therefore, even in a case where the cord-shaped piezoelectric sensors are disposed in a wave-shaped fashion at the lower portion of the bed, the sensors are placed in on states at the moment where they are bent but do not output signals after the completion of the disposition. Then, thereafter, each of the cord-shaped piezoelectric sensors outputs a signal only when a force is applied to a portion thereof

Next, the construction and function of the bed will be explained more in detail.

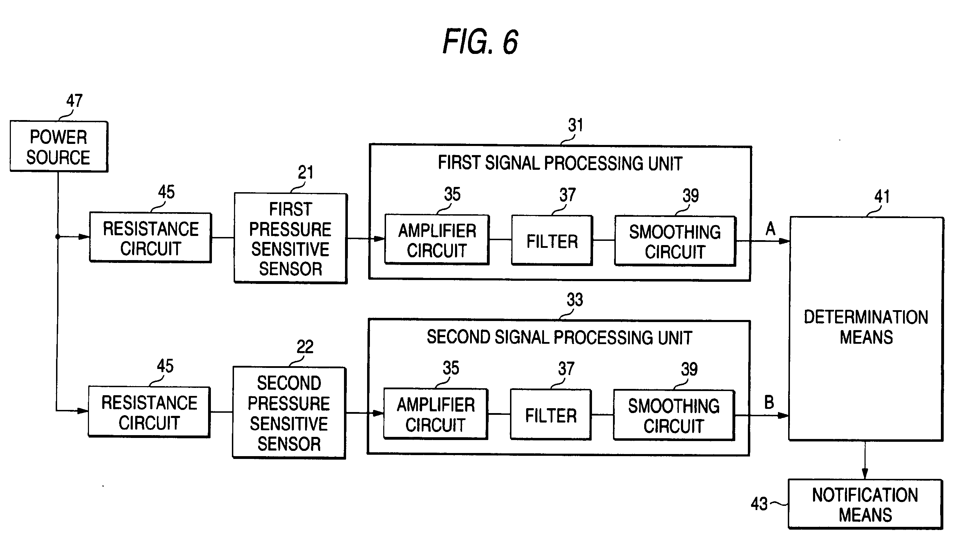

Fig. 6 is a block diagram showing the construction and function of the detection device of the bed.

One and the other of a pair of the pressure sensitive sensors 21, 22 provided at the pressure sensitive sheet 13 serve as a first pressure sensitive sensor 21 and a second pressure sensitive sensor 22, respectively.

The first pressure sensitive sensor 21 is coupled to a first signal processing unit 31 and the second pressure sensitive sensor 22 is coupled to a second signal processing unit 33. In the signal processing units 31, 33, output signals from the pressure sensitive sensors 21, 22 are applied to amplifier circuits 35, respectively.

Each of the first signal processing unit 31 and the second signal processing unit 33 is arranged in a manner that a filter 37 and a smoothing circuit 39 are provided at the rear stage of the amplifier circuit 35, and that the output signal from the corresponding one of the first pressure sensitive sensor 21 and the second pressure sensitive sensor 22 is amplified by the amplifier circuit 35, then a required frequency component is filtered by the filter 37 and the signal waveform is smoothed by the smoothing circuit 39.

Then, each of the first signal processing unit 31 and the second signal processing unit 33 is coupled to a determination means 41. Electric signals smoothed by the smoothing circuits 39 are inputted into the determination means 41.

The determination means 41 determines the shift of the position of a bedded person lying on the bedding surface 15a of the mattress 15 based on the electric signals supplied from the first signal processing unit 31 and the second signal processing unit 33.

Further, a notification means 43 is coupled to the determination means 41. The notification means 43 is constituted by a sound output means such as a speaker or a display means such as a display. The determination means 41 outputs a notification signal to the notification means 43 based on the determination result of the positional shift of a bedded person. Then, the notification means 43 outputs an alarm by means of sound or display or the combination thereof in response to the reception of the notification signal from the determination means 41 thereby to notify that the position of a bedded person on the bedding surface 15a is shifted.

Incidentally, each of the first pressure sensitive sensor 21 and the second pressure sensitive sensor 22 is coupled to a power source 47 via a resistance circuit 45 and is supplied with a driving voltage for detection via the resistance circuit 45 from the power source 47 in advance.

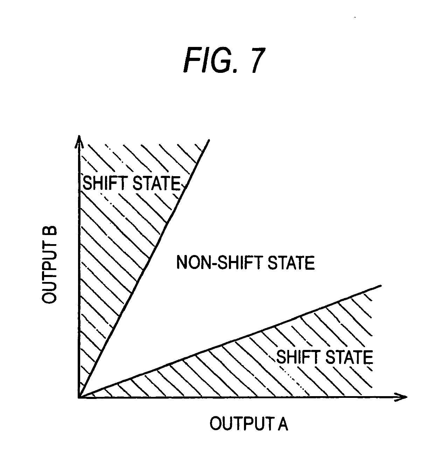

The determination means 41 determines the positional shift of a bedded person based on a ratio between the intensities (using the integration intensities etc. of the amplitudes or peaks of the signals) of the electric signals from the first signal processing unit 31 and the second signal processing unit 33. To be concrete, as shown in Fig. 7, a table is prepared in advance which determines a range of the shift concerning the ratio of the intensities of the outputs A, B of the electric signals from the first signal processing unit 31 and the second signal processing unit 33. Then, the outputs A, B of the electric signals from the first signal processing unit 31 and the second signal processing unit 33 are compared and it is determined whether the ratio of the intensities of the outputs A, B is in the range of a predetermined shift state or within a non-shift state.

Then, when the ratio of the intensities of the outputs A, B is in the range of the shift state away from the non-shift state, the determination means 41 determines that the position of a bedded person on the bedding surface 15a is shift and so outputs the notification signal to the notification means 43.

The notification means 43, in response to the notification signal, outputs an alarm to a third party such as a care person by means of sound or display or the combination thereof thereby to notify that the position of a bedded person on the bedding surface 15a is shifted.

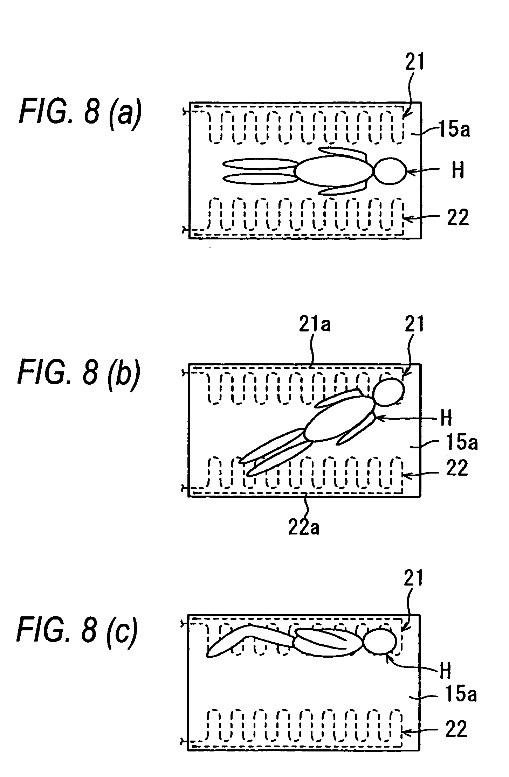

In the bed provided with the detection device for a bedded person configured in the aforesaid manner, an adjustment is made prior to the detection of the shift in order to correct a personal difference among bedded persons, whereby the detection accuracy can be improved largely. As a concrete adjustment process, firstly, as shown in Fig. 8 (a), in a state where a bedded person H is laid between the first pressure sensitive sensor 21 and the second pressure sensitive sensor 22, a not-shown adjusting knob provided at the determination means 41 is rotated thereby to adjust so as to make the intensity of the output A from the first signal processing unit 31 almost coincide with the intensity of the output B from the second signal processing unit 33.

In a state after this adjustment, when a bedded person H on the bedding surface 15a moves on the bedding surface 15a as shown by Fig. 8(b) or (c), the loads applied to the first pressure sensitive sensor 21 and the second pressure sensitive sensor 22 change. According to the changes of the loads, the pushing forces of the projection pieces of the projection sheet 11 against the pressure sensitive sensors also change largely, so that the first pressure sensitive sensor 21 and the second pressure sensitive sensor 22 output the detection signals. In the case of Fig. 8(b), the head portion of a bedded person H is placed on the first pressure sensitive sensor 21 and the foot portion of the bedded person is placed on the second pressure sensitive sensor 22. Since the head portion is heavier than the foot portion, the head portion sinks lower in the mattress 15, and so the output of the pressure sensitive sensor at the head portion becomes larger than that of the output of the pressure sensitive sensor at the hoot portion. As a result, the difference between the signal intensities of the outputs A and B becomes larger. Thus, the shift state of a bedded person can be detected. In this manner, the shift state can be detected due to the large load change at the time where a bedded person H is in an shift state. Further, even when a bedded person had shifted in a shift state and has been in a static state, the vibration is detected by the pressure sensitive sensors 21, 22 due to a small movement of the body caused by the breathing or heartbeat. Thus, it is possible to determine whether a bedded person is in a shift state or not by comparing the magnitude of the vibration.

Further, in particular, in a shift state shown in Fig. 8(c) in which a bedded person may fall with a high possibility, the difference between the intensities of the output signals of the pressure sensitive sensors 21, 22 becomes large particularly. When the notification level is classified in accordance with the magnitude of the intensity difference of the output signals, it is possible to notify in a manner of distinguishing the case where an urgent countermeasure is required.

Furthermore, in this case, a tensile force, which is caused by the sinking of the mattress 15 due to the weight of a bedded person H, acts at the straight line portions 21 a, 22a of the first and second pressure sensitive sensors 21, 22, whereby the detection signal is also generated. Thus, the detection sensitivity is further improved.

In this manner, according to the detection device and the detection method for a bedded person described above, the first pressure sensitive sensor 21 and the second pressure sensitive sensor 22 each configured by the cable-shaped piezoelectric sensor are disposed along the side edges of the bedding surface 15a at the both end sides of the bedding surface 15a, respectively, and the determination means 41 compares the output signals from the first pressure sensitive sensor 21 and the second pressure sensitive sensor 22 thereby to determine the positional shift to the side portion of a bedded person on the bedding surface 15a. Thus, the bedding state of a bedded person can be grasped from the determination result and so a bedded person can be prevented from falling etc. in advance from the bedding surface 15a.

Further, since the cable-shaped piezoelectric sensors are merely disposed at the both end sides of the bedding surface 15a, the increase of the cost can be suppressed utmostly as compared with a case where many sensors each configured by an expensive piezoelectric element are disposed in a lattice fashion with a space therebetween in the width direction and the longitudinal direction of the bed.

Further, since each of the pressure sensitive sensors 21, 22 itself is a sensor with a quite high sensitivity, a sufficient detection sensitivity can be obtained even when the pressure sensitive sheet 13 provided with the pressure sensitive sensors 21, 22 is disposed beneath the mattress 15, so that it is possible to eliminate such a problem that the pressure sensitive sensors 21, 22 badly influence on the comfortability of the bed. Furthermore, even in the case where the pressure sensitive sensors 21, 22 are disposed just under the bedding surface 15a, since the pressure sensitive sensors 21, 22 are not disposed at the center portion of the bedding surface 15a and the pressure sensitive sensors 21, 22 themselves have flexibility, the degradation of the comfortability of the bed can be suppressed utmostly.

Furthermore, the detection sensitivity of the pressure sensitive sensors 21, 22 can be improved quite easily by merely laminating the pressure sensitive sheet 13 provided with the pressure sensitive sensors 21, 22 and the projection sheet 11 provided with the projection pieces 17.

Incidentally, in the aforesaid embodiment, although the sensitivity is intended to be improved by laminating the projection sheet 11 provided with the projection pieces 17 on the pressure sensitive sheet 13 provided with the pressure sensitive sensors 21, 22, the structure for improving the sensitivity maybe configured in the aforesaid manner.

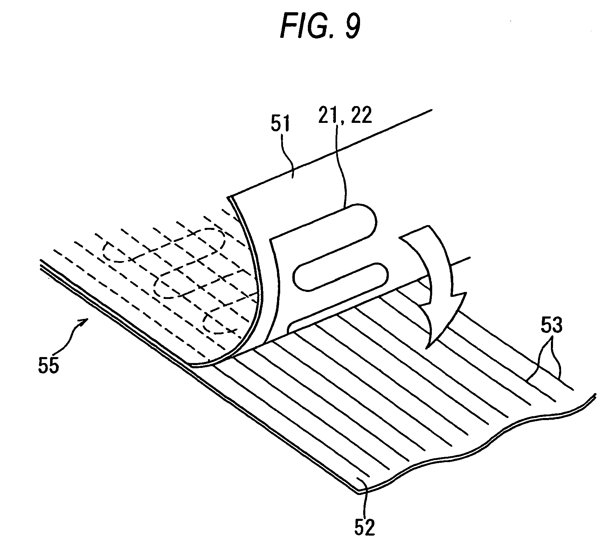

The configuration shown in Fig. 9 is arranged in a manner that as to two sheets 51, 52 each formed by non woven cloth etc., for example, the pressure sensitive sensors 21, 22 are adhered to the one sheet 51, projection pieces 53 each formed by a wire rod etc. are formed on the other sheet 52, and these sheets 51, 52 are laminated thereby to constitute a pressure sensitive sheet 55. In the case of using the pressure sensitive sheet 55, the aforesaid projection sheet 11 can be eliminated and so the configuration can be simplified. Also, according to this configuration, the projection pieces 53 serve to act the load from a bedded person on the pressure sensitive sensors 21, 22 as a large force locally, so that the sensitivity can be improved. Of course, the projection pieces 53 may be disposed at either above or beneath the pressure sensitive sensors 21, 22.

The heart beat of a bedded person may be detected by the pressure sensitive sensors 21, 22, and the health condition of the bedded person can be managed based on the detection result.

Next, the explanation will be made as to a modified example of the pressure sensitive sheet of bed according to the first embodiment

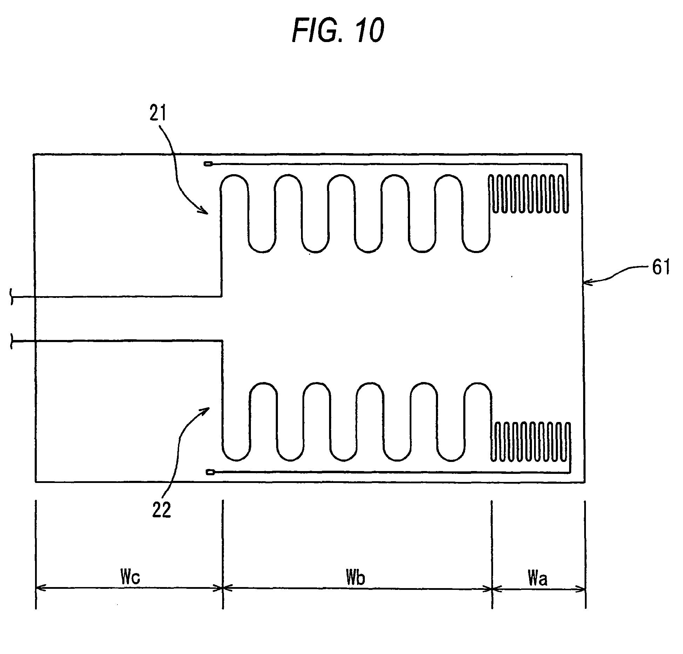

Fig. 10 is a schematic plan view showing the modified example of the pressure sensitive sheet of the first embodiment.

A pressure sensitive sheet 61 is arranged in a manner that the interval of the adjacent waves of the wave-shaped portion of each of the pressure sensitive sensors 21, 22 is made shorter thereby to increase the wiring density in a region Wa which is the head portion side of a bedded person laying on the bedding surface 15a. In contrast, the interval of the adjacent waves of the wave-shaped portion of each of the pressure sensitive sensors 21, 22 is made longer thereby to decrease the wiring density in a region Wc which is the leg portion side of a bedded person laying on the bedding surface 15a (the figure shows an example where the pressure sensitive sensors are made linear in a bedding direction thereby to minimize the wiring density.

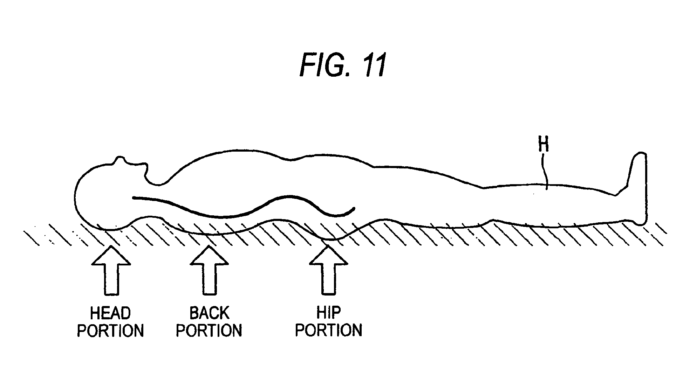

In general, as shown in Fig. 11, when a bedded person lies, a large load is applied to the bedding surface 15a at the head portion, the back portion and the hip portion, whilst not a large load is applied at the leg portion. In particular, the positional shift of a bedded person is largely influenced by the positional shift of the head portion. Thus, in this embodiment, the pressure sensitive sensors 21, 22 are arranged so as to be high in the wiring density on the head portion side so that even a small movement can be detected, whilst arranged so as to be low in the wiring density on the leg portion side at which the movement is relatively frequent thereby to likely cause an erroneous detection.

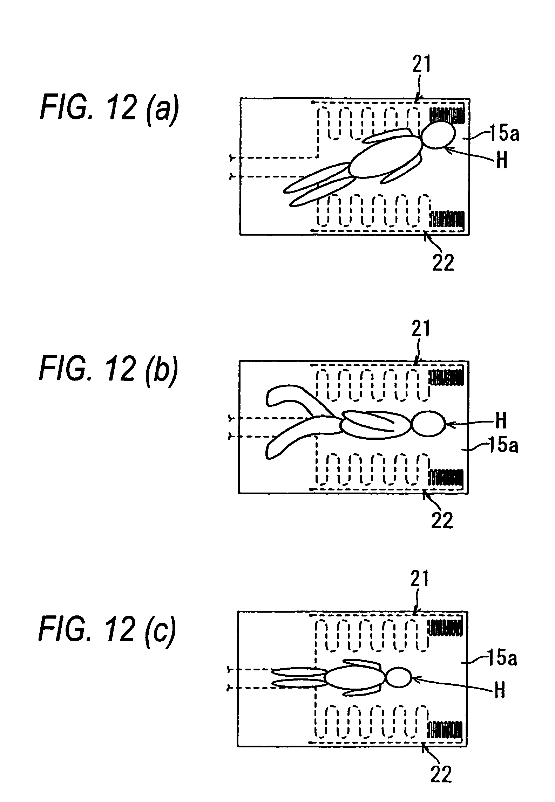

In the detection device for a bedded person according to the embodiment, as shown in Fig. 12(a), even when such a shift arises that a bedded person H lies on the bedding surface 15a in a slanted manner so as to slightly lie above the pressure sensitive sensor 21 in the region Wa, the positional shift of the head portion of the bedded person H can be surely detected with a high sensitivity.

Further, since the pressure sensitive sensors 21, 22 are arranged so as to be low in their density on the leg portion side, as shown in Fig. 12(b), even when an shift state does not occur such that a bedded person H lies at the center portion on the bedding surface 15a with the person's leg being opened, it is possible to eliminate such a problem that an alarm is unnecessarily issued by the reason that the position of the bedded person H is shifted.

Further, as shown in Fig. 12(c), when a bedded person H is light-weighted and small like a child etc., the detection sensitivity of the pressure sensitive sensors 21, 22 becomes low. However, since the pressure sensitive sensors 21, 22 on the leg portion side are disposed closer to the center portion, the detection of a bedded person H can be performed surely. Thus, the existence of a bedded person can be surely detected and further the positional shift of a bedded person can be detected with a high accuracy like the aforesaid case.

The pressure sensitive sensors 21, 22 in the respective embodiments are particularly desired to be piezoelectric sensors configured by the aforesaid material. However, in the invention, the pressure sensitive sensors are not limited to such sensors but various kinds of pressure sensitive sensors such as an electric contact type cable-shaped sensor may be used which is arranged in a manner that, for example, a pair of electric contacts are buried within a cable and the contacts are made contact to each other due to the bending of the cable thereby to detect a pressure.

### Embodiment 2

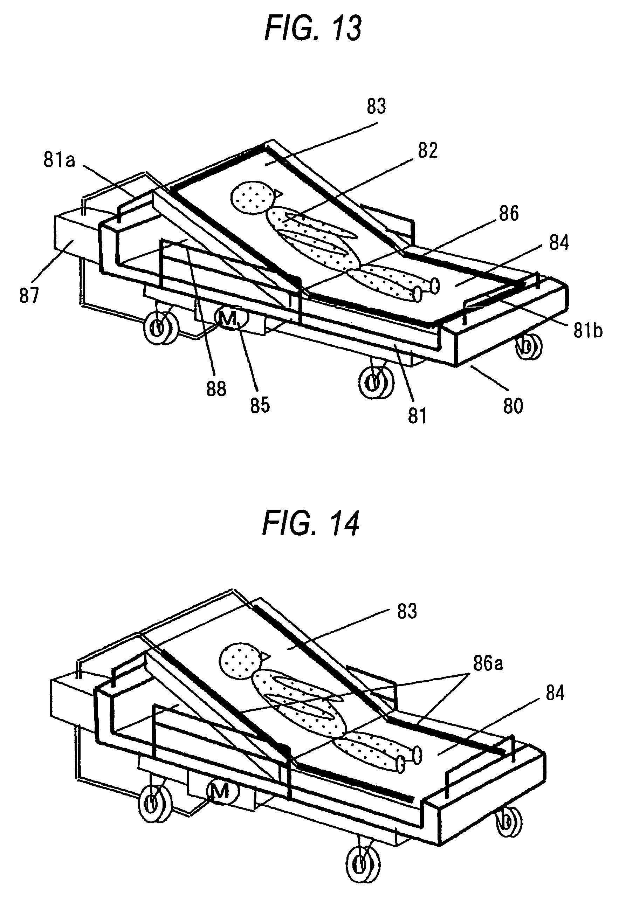

The second embodiment of the invention will be explained with reference to Figs. 13 to 27.

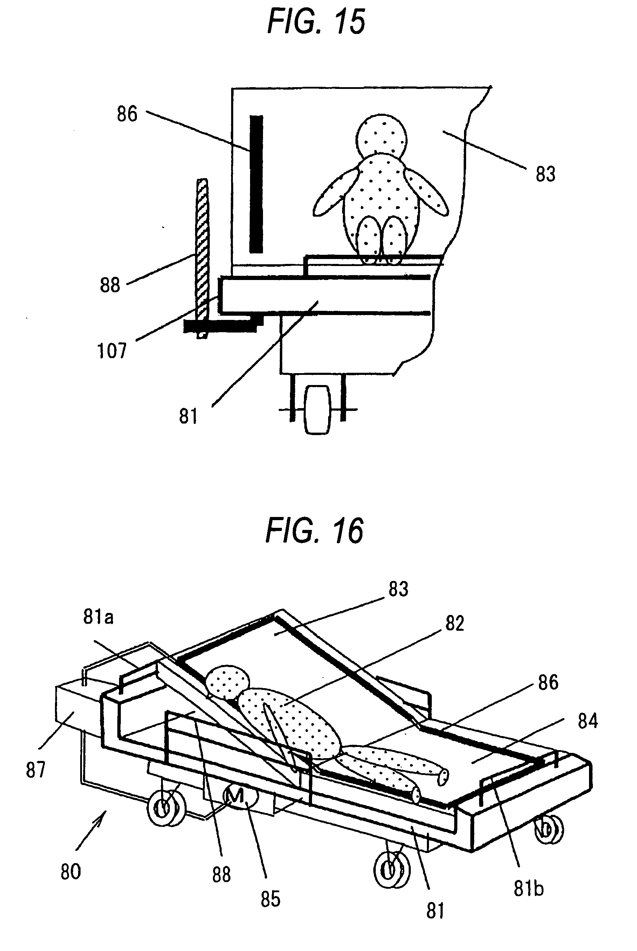

The second embodiment of the invention is explained based on Figs. 13 to 27. Fig. 13 is an external perspective view of an electrically driven bed in which a pressure sensitive sensor is mounted. Fig. 14 is an external perspective view of the apparatus in which pressure sensitive sensors are disposed at portions of the surface end portions of a back raising panel portion and a knee raising panel portion. Fig. 15 is a schematic side view showing the positional relation between the bed and the side fence of the apparatus. Fig. 16 is a schematic perspective view showing a state where a bedded person touches the pressure sensitive sensor.

As shown in Fig. 13, the electrically driven bed 80 serving as a bed according to the second embodiment is arranged in a manner that the back raising panel portion 83 serving as a panel portion for a portion corresponding to the upper part of a bedded person 82 and the knee raising panel portion 84 that is a portion corresponding to the lower part of the bedded person are mounted on a bed frame 81. The bed frame 81 includes a raising frame 81a provided at the upper end side of the back raising panel portion 83 and a raising frame 81b provided at the lower end side of the knee raising panel portion 84. Further, the apparatus includes a driving means 85 for raising the upper part and the lower part of the bedded person 82. The pressure sensitive sensor 86 generates an output signal according to a deformation thereof caused by a pressing load applied when the bedded person 82 touches the sensor. To this end, the pressure sensitive sensor is dispose along the entire periphery of the surface end portions of the back raising panel portion 83 and the knee raising panel portion 84 and further along the side edges of the back raising panel portion 83 and the knee raising panel portion 84. A determination means 87 is provided which determines whether or not the bedded person 82 touches the pressure sensitive sensor 86 based on the output signal according to the deformation of the pressure sensitive sensor 86. The elevational driving operation of the driving means 85 is controlled by a control means (not shown) based on a determination signal from the determination means 87. The electrically driven bed 80 is provided with side fences 88 at the both sides along the width direction of the bed frame 81.

As shown in Fig. 14, the pressure sensitive sensor may be configured so as to be disposed at the portion of the surface end portions of the back raising panel portion and the knee raising panel portion. This configuration is more practical since the length of the pressure sensitive sensor can be made shorter as compared with the configuration in which the sensor is provided at the entire periphery.

Fig. 15 shows the positional relation between the bed and the side fence in the electrically driven bed. Between the side fence 88 and the bed frame 81 or the side surface 107 of the back raising panel portion 83 and the knee raising panel portion 84 as the bed, a small distance is structurally required in order to attach the side fence 88. Thus, at the time of elevating the back raising panel portion 83, there may arise a case that a subject is caught in the space inadvertently. Although the side fence 88 is provided in order to prevent the bedded person 82 from falling from the bed, the safety control is required in combination with the electrically driven bed.

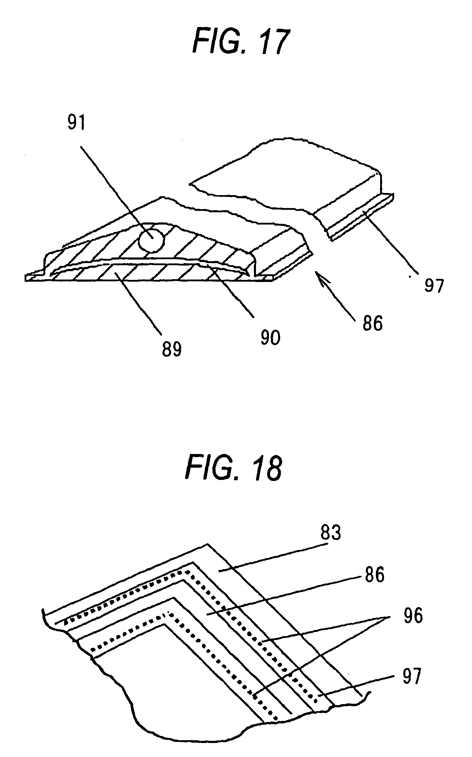

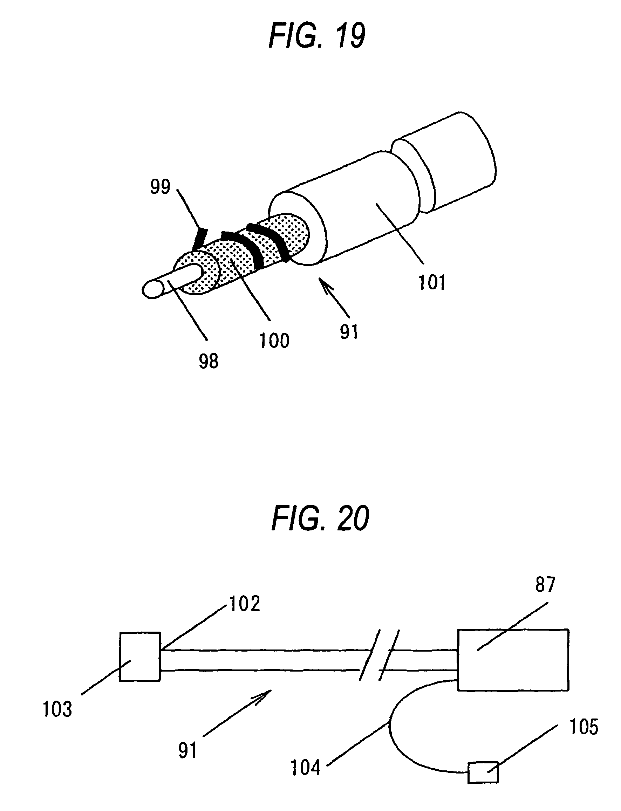

Fig. 17 is an external view of the pressure sensitive sensor. Fig. 18 is a schematic diagram of a main portion showing a state where the pressure sensitive sensor is fixed to the back raising panel portion and the knee raising panel portion by means of a sewing machine. Fig. 19 is a perspective view of a main portion of the piezoelectric sensor serving as the pressure sensitive sensor. Fig. 20 is an external view of a system of the piezoelectric sensor serving as the pressure sensitive sensor having a determination means.

In Fig. 17, the pressure sensitive sensor 86 (including 86a but hereinafter merely referred to 86) is provided with a deformation means 89 formed by elastic member, a non-linear flexible member 90 and a piezoelectric sensor 91 of a flexible cable shape. As the non-linear flexible member 90, a belt-shaped thin-type elastic member of a convex shape used in a convex measure, for example, is employed. Such a belt-shaped thin-type elastic member has characteristics that it deforms suddenly in a concave shape when a pressing force load applied thereto becomes a predetermined value or more and restores to the original shape when the pressing force load is removed.

The sensor shown in Fig. 18 is arranged in a manner that the attachment flange portion 97 of the pressure sensitive sensor 86 is fixedly integrated by means of the sewing 96 using a sewing machine at the surface end portions of the back raising panel portion 83 and the knee raising panel portion 84. The pressure sensitive sensor 86 may be configured in a manner that it is fixedly integrated by means of a double-stick tape or an adhesive etc. to the back raising panel portion 83 and the knee raising panel portion 84.

Fig. 19 is a diagram showing the configuration of the main portion in which a part of a flexible cable-shaped piezoelectric sensor 91 according to the first embodiment of the invention.

The piezoelectric sensor 91 is configured in a manner that a center electrode 98 serving as an electrode for extracting a signal, an outer electrode 99, a composite piezoelectric layer 100 made of composite electric material formed by mixing sintered powder of piezoelectric ceramics into rubber elastic material, and a cover layer 101 are laminated concentrically, then formed in a cable shape and subjected to the polarizing processing. This piezoelectric sensor has the similar configuration as that shown in Fig. 5 and has excellent flexibility and generates an output signal according to the elastic deformation thereof As the piezoelectric ceramics, sintered powder of lead based piezoelectric material such as lead titanate or titanate lead zirconate is used or sintered powder of non-lead based piezoelectric material such as sodium niobate is used. As the center electrode 98, although normal metal single line may be used, in this case an electrode which is formed by winding a meal coil around the circumferential periphery of an insulative macromolecular fiber is used. As the insulative macromolecular fiber and the metal coil, polyester fiber and copper alloy containing 5 wt% of silver commercially used in an electric heating blanket are desirable, respectively. Further, as the outer electrode 99, a belt-shaped electrode which is formed by adhering a metal film on a macromolecule layer is used. The outer electrode is configured in a manner that the belt-shaped electrode thus formed is wound around the circumferential periphery of the composite piezoelectric layer 100. Polyethylen terephthalate (PET) is used as the macromolecule layer. The belt-shaped electrode which is formed by adhering an aluminum film on the macromolecule layer has high heat stability at 120 C° and is mass-produced commercially, so that the belt-shaped electrode is preferable as the outer electrode 99.

Incidentally, in order to shield from the electric noises in the external environment, the piezo electric sensor 22 serving as the pressure sensitive sensor 86 is preferably arranged in a manner that the outer electrode 99 is wound around the circumferential periphery of the composite piezoelectric layer 100 so as to be partially overlapped thereon. Although vinyl chloride or polyethylene may be used as the cover layer 101, elastic material such as rubber having better flexibility and elasticity than the composite piezoelectric layer 100 may be used so that the piezoelectric sensor 91 likely deforms elastically when a bedded person 82 touches the sensor. As such rubber, for example, ethylene propylene rubber (EPDM), chloroprene rubber (CR), butyl rubber (IIR), silicon rubber (Si) or thermoplastic elastomer may be used.

Fig. 20 is an external view of the system of the piezoelectric sensor 91 serving as the pressure sensitive sensor 86 having a determination means 87. In Fig. 20, a wire-breakage detection resistor 103 is contained within an one end portion 102 of the cable-shaped piezoelectric sensor 91. The wire-breakage detection resistor 103 is coupled between the center electrode 98 of the cable-shaped piezoelectric sensor 91 and the outer electrode 99. The wire-breakage detection resistor 103 also serves as a discharge portion for discharging electric charges that are generated at the piezoelectric sensor 91 by the pyroelectric effect, and so parts can be rationalized. The determination means 87 for determining the touch of a bedded person 82 to the sensor is directly coupled to the piezoelectric sensor 91, whereby the piezoelectric sensor 91 and the determination means 87 are integrated. 104 depicts a cable for supplying electric power and outputting a detection signal, and 105 depicts a connector.

Next, the operation and function of the aforesaid configuration will be explained.

As shown in Fig. 16, in a case where a bedded person 82 changes the posture from a state laying on one's back to a state laying on one's side due to any reason during the elevational operation of the back raising panel portion 83 and the knee raising panel portion 84 and so the person shifts from the almost center position of the back raising panel portion 83 and the knee raising panel portion 84 to the end position, if the bedded person 82 touches the pressure sensitive sensor 86, the output signal from the pressure sensitive sensor 86 is transmitted to the determination means 87, so that the determination means 87 determines that the bedded person 82 touches the sensor. Then, the drive means is controlled by the control means (not shown) thereby to immediately stop the elevational driving of the back raising panel portion 83 and the knee raising panel portion 84. In this case, the back raising panel portion 83 and the knee raising panel portion 84 may be controlled so as to be restored to their original positions. In such a case, if the body of a bedded person is caught at the time of stopping the driving operation, since the caught state can be released, the safety of the apparatus can be further improved.

The aforesaid operation control is performed only when the back raising panel portion 83 and the knee raising panel portion 84 are during the elevational driving but not performed during the non-driving state of the back raising panel portion 83 and the knee raising panel portion 84 even if the pressure sensitive sensor 86 detects the movement of a bedded person 82.

Further, in a case where a part of the body of a bedded person 82 protrudes from the upper end of the back raising panel portion 83 due to any reason during the elevational operation of the back raising panel portion 83, if the part of the body touches the pressure sensitive sensor 86, the output signal from the pressure sensitive sensor 86 is transmitted to the determination means 87, so that the determination means 87 determines that the bedded person 82 touches the sensor. Then, the elevational driving of the back raising panel portion 83 and the knee raising panel portion 84 is immediately stopped. Thus, it is possible to prevent such a problem that a part of the body abuts against the raising frame 81a inadvertently and a part of the body is caught by the raising frame 81a inadvertently.

Furthermore, in a case where a part of the body of a bedded person 82 protrudes from the lower end of the knee raising panel portion 84 due to any reason during the elevational operation of the knee raising panel portion 84, if the part of the body touches the pressure sensitive sensor 86, the output signal from the pressure sensitive sensor 86 is transmitted to the determination means 87, so that the determination means 87 determines that the bedded person 82 touches the sensor. Then, the elevational driving of the knee raising panel portion 84 is immediately stopped. Thus, it is possible to prevent such a problem that a part of the body abuts against the raising frame 81b inadvertently and a part of the body is caught by the raising frame 81 b inadvertently.

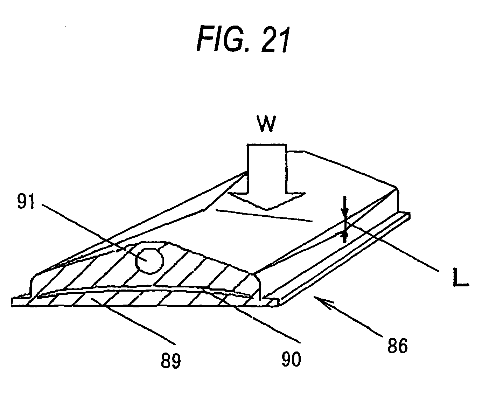

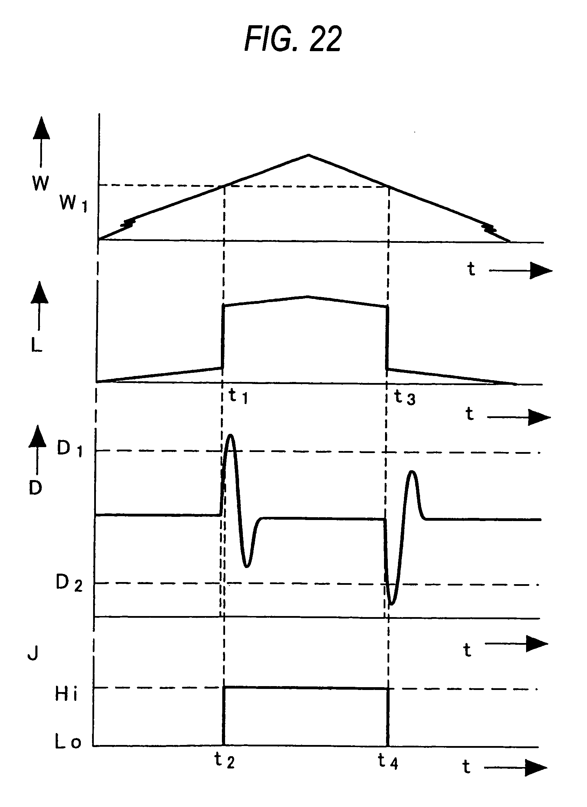

Fig. 21 is an external view showing a state of the deformation of the pressure sensitive sensor 86 when a load W is applied to the pressure sensitive sensor 86 due to the touch of a bedded person 82 to the sensor. Fig. 22 is a characteristic diagram showing the load W, the displacement L (see Fig. 21) of the pressure sensitive sensor 86, the output signal D of the piezoelectric sensor 91 and the determination output J of the determination means. In Fig. 22, the ordinate represents W, L, D and J from the top, whilst the abscissa represents the time t.

In Figs. 21 and 22, when a load W is applied to the pressure sensitive sensor 86 due to the touch of a bedded person 82 thereto and then W becomes larger than W1 at a time point t1, the non-linear flexible member 90 deforms in a concave shape and so L increases rapidly in a non-linear manner. The state in this case is shown in Fig. 21. In this case, since the piezoelectric sensor 91 also deforms largely, a large signal appears as D. Then, the determination means 87 determines that the bedded person 82 touches the sensor when D is larger than D1 thereby to set J to a high level (Hi) at a time point t2 and keep the level. Then, W is reduced gradually, and when W becomes smaller than W 1 at a time point t3, the non-linear flexible member 90 deforms in a convex shape and restores to the original shape, whereby L decreases rapidly in a non-linear manner. In this case, since the piezoelectric sensor 91 also deforms largely in the direction opposite to the aforesaid direction, a large signal having the polarity opposite to that of the aforesaid case appears as D. Then, the determination means 87 determines that the bedded person 82 detaches from the sensor when D is smaller than D2 thereby to set J to Lo at a time point t4.

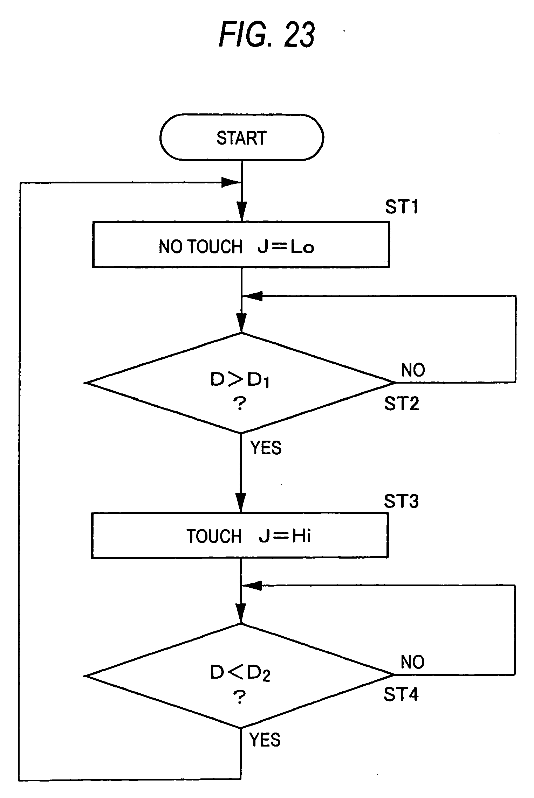

Fig. 23 is a flowchart showing the aforesaid determination procedure. At first, when the operation is started, J is set to Lo representing no-touch by a bedded person 82 as the initial value in step ST1. When D is determined to be larger than D1 in step ST2, it is determined that the bedded person 82 touches the sensor in step ST3, and so J is set to Hi. In contrast, when D is determined to be equal to or smaller than D1, it is waited until D becomes larger than D1 in step ST2. Next, when D is determined to be smaller than D2 in step ST4, the process returns to step ST1, and it is determined that the bedded person 12 detaches from the sensor thereby to set J to Lo. In contrast, when D is determined to be equal to or larger than D2, it is waited until D becomes smaller than D2 in step ST4.

The reason why the combination of the non-linear flexible member and the piezoelectric sensor with flexibility is used as the pressure sensitive sensor according to the embodiment is as follows.
(1) In the case where, as a pressure sensitive sensor of a type for detecting a load, a tape-shaped pressure sensitive sensor of an electrode contact type or a pressure sensitive sensor of a pressure-sensitive resistance change type which resistance value changes depending on a load, each of which being used normally, is disposed at the periphery of the mattress of the bed, when the back raising panel portion or the knee raising panel portion is elevationally driven, the pressure sensitive sensor causes an erroneous detection due to a stress generated by the bending thereof at the portion where the mattress bends.
(2) In order to solve the aforesaid problem, when the pressure sensitive sensor is divided and the divided pieces thereof are disposed separately so as to avoid the bent portion, even when a subject contacts with the pressure sensitive sensor, the mattress absorbs the pressing force due to the softness of the mattress, whereby there arises a case that the contact can not be detected. In particular, in the case of employing a bed using a mattress of low-repulsion urethane foam being excellent in body pressure distribution property for preventing bedsore, such non-detection likely occurs.

In contrast, the pressure sensitive sensor of the embodiment uses the piezoelectric sensor with flexibility and generates the output signal according to the acceleration of the deformation. The operation speed of the elevational driving of the back raising panel portion and the knee raising panel portion is made low in view of the safety. Thus, the acceleration of the deformation of the pressure sensitive sensor caused at the bent portion of the mattress as described above becomes also small, so that the output signal from the piezoelectric sensor generated by the elevational driving can be suppressed to a small value. As a result, it is possible to avoid the erroneous determination by suitably selecting a threshold value for determining the contact in the determination mean.

Further, since the non-linear flexible member is also used, even if the mattress is soft and the contact speed is slow, the non-linear flexible member deforms abruptly when a load of the predetermined value or more is applied thereto due to the contact with a subject. Thus, since the piezoelectric sensor also deforms abruptly due to such a displacement of the non-linear flexible member, the output signal having a magnitude sufficient for detecting the contact with a subject can be obtained from the pressure sensitive sensor.

As described above, the embodiment is arranged to control the pressure sensitive sensor disposed at the entire periphery or the part of the surface end portion of at least one of the back raising panel portion and the knee raising panel portion, the determination means for determining the touch of a bedded person to the pressure sensitive sensor based on the output signal from the pressure sensitive sensor, and the driving means for elevationally driving the back raising panel portion and the knee raising panel portion in accordance with the determination signal from the determination means. Thus, when it is determined that a bedded person contacts with the pressure sensitive sensor during the elevational operation of the back raising panel portion or the knee raising panel portion, such a control can be performed that the elevational driving of the back raising panel portion or the knee raising panel portion is stopped or the driving direction is reversed. As a result, an inadvertent caught at the time of the elevational movement of the bed can be prevented.

Furthermore, since the pressure sensitive sensor is configured by the non-linear flexible member which displacement amount with respect to a load is non-linear and the piezoelectric sensor with flexibility which deforms according to the displacement of the non-linear flexible member, the sensitivity relating to the contact of a bedded person to the pressure sensitive sensor can be enhanced. This is because when the pressing load of a bedded person against the pressure sensitive sensor is the predetermined value or more, the non-linear flexible member deforms abruptly. Thus, since the piezoelectric sensor also deforms abruptly due to such a displacement of the non-linear flexible member, the output signal having a magnitude sufficient for detecting the contact with a subject can be obtained from the pressure sensitive sensor.

Further, since the non-linear flexible member is configured by a thin type elastic material which is formed in a belt shape and has a convex portion, it is simple and highly practical.

Furthermore, since the non-linear flexible member and the piezoelectric sensor are disposed in the deformation means capable of deforming according to a load, both the non-linear flexible member and the piezoelectric sensor are further apt to deform when a load is applied thereto, so that the sensitivity of the pressure sensitive sensor can be further improved.

Furthermore, the control means is arranged to validate the determination signal from the determination means when the driving means performs the elevational driving and to invalidate the determination signal from the determination means when the driving means does not perform the elevational driving. Accordingly, even if a bedded person or a third party touches the pressure sensitive sensor during the stationary state of the back raising panel portion, for example, the back raising panel portion is prevented from being inadvertently driven elevationally. Since the determination signal from the determination means is made valid only when the back raising panel portion is driven elevationally, it is possible to provide the electrically driven bed which provides the sense of security and does not perform erroneous operation.

Incidentally, concerning the embodiment, although the description is made as to the electrically driven bed in which the back raising panel portion 83 and the knee raising panel portion 84 are driven elevationally, the pressure sensitive sensor having the same configuration as the pressure sensitive sensor 86, 86a may be applied to the electrically driven bed in which only the back raising panel portion 83 is driven elevationally.

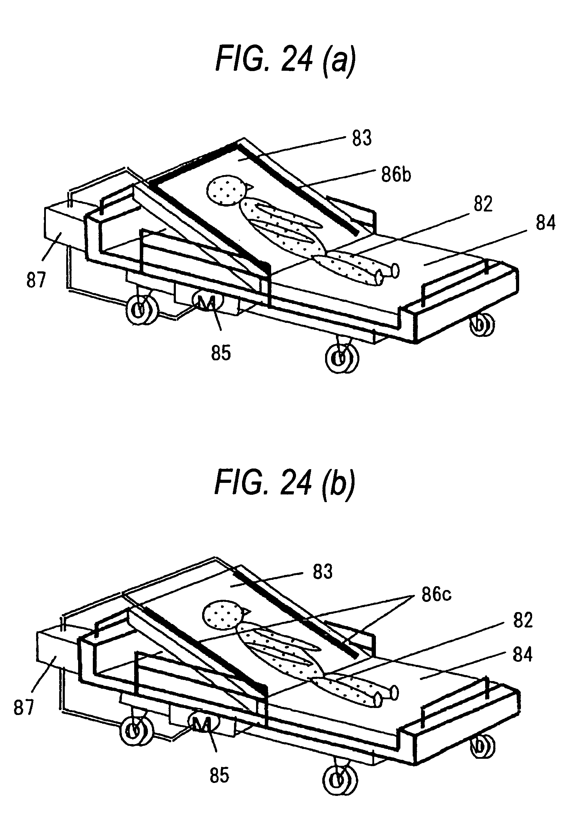

The arrangement of the pressure sensitive sensor with this configuration will be explained based on Fig. 24. Fig. 24(a) shows a case where the pressure sensitive sensor 86b is disposed in a U-shape manner on the surface of the back raising panel portion along the side edges at the upper end portion and the both end portions in the width direction of the back raising panel portion 83. Fig. 24(b) shows a case where pressure sensitive sensors 86c are disposed on the surface of the back raising panel portion along the side edges at the both end portions in the width direction of the back raising panel portion 83. The determination means 87 is provided which determines the contact of a bedded person 82 to the pressure sensitive sensor 86b or 86c based on the output signal from the pressure sensitive sensor 86b or 86c. Thus, when the back raising panel portion 83 is controlled so as to stop its elevational driving or be restored to its original position in accordance with the determination result of the determination means 87, it is possible to provide the electrically driven bed which can prevent an inadvertent caught of a bedded person.

Further, it may be arranged in a manner that the pressure sensitive sensors formed by the piezoelectric sensors are disposed at the both sides of the mattress so that the pressure sensitive sensors detect the shift of the laying position of a bedded person on the mattress, and when the shift is determined to exist, the elevational driving is inhibited thereby to prevent the caught in advance. In this case, the shift is determined in a manner that, for example, each of the piezoelectric sensors detects a small movement of the body based on the heartbeats or breathing of a bedded person, and it is determined that the shift exists when a ratio of the output signals of the piezoelectric sensors based on the small movement of the body is a setting value or more. That is, the output signals of the piezoelectric sensors based on the small movement of the body are used in a manner that it is determined that the shift exists when an amplitude of the output signal of one of the piezoelectric sensors is a predetermined times or more as large as the amplitude of the output signal of the other of the piezoelectric sensors. When it is determined that the shift exists, an alarm may be issued so as to notify that a bedded person is not safety due to the shift and a message may be issued so as to urge the bedded person to return to the center of a bed. Alternatively, the existence of the shift may be notified to a care person by means of a nurse call system or a communication means. Further, when both this configuration and the configuration of the second embodiment are employed, the caught can be prevented more effectively.

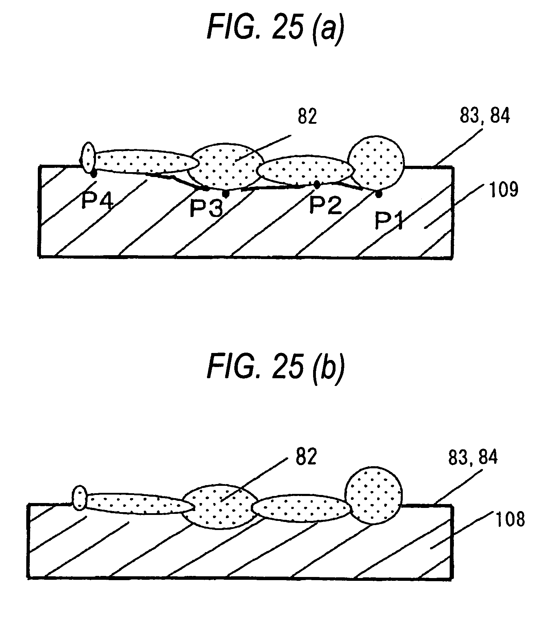

Incidentally, although it is possible to detect a small movement of the body based on the heartbeats or breathing of a bedded person by using the mattress formed by usual urethane foam, the small movement of the body based on the heartbeats or breathing of a bedded person can be detected with better sensitivity by using the low-repulsion urethane foam. This will be explained based on Fig. 25. Fig. 25(a) is a sectional view showing a case where a person is sleeping by using the usual urethane foam and Fig. 25(b) is a sectional view showing a case where a person is sleeping by using the low-repulsion urethane foam.

The remarkable difference of the physical property value between the usual urethane foam and the low-repulsion urethane foam is that the rebound resilience value of the former is about 35% but that of the latter is about 5%. In other words, the usual urethane foam has a larger repulsion property. This will be described based on the experimental verification results performed by the applicant etc. of the invention. In the case of the usual urethane foam 109, as shown in Fig. 25(a), the convex portions on the back side of a bedded person 82, that is, a head portion P1, a scapula portion P2, a hip portionP3 and heel portions P4 particularly abut strongly against the back raising panel portion 83 and the knee raising panel portion 84 of the bed and so sink lower.

In contrast, in the case of the low-repulsion urethane foam 108, as shown in Fig. 25(b), the portions of the back side of a bedded person 82 such as a neck, s shoulder, a back, a waist, a hip, thighs, calves, heels of legs etc. from a head side sink uniformly against the back raising panel portion 83 and the knee raising panel portion 84 of the bed.

Due to such a difference of the characteristics, in the case of the usual urethane foam 109, since the body partially contacts with the mattress, the small movement of the body based on heartbeats or breathing is hardly transmitted to the mattress. As a result, since the small movement of the body is hardly transmitted to the piezoelectric sensor, the displacement of the piezoelectric sensor due to the small movement of the body is small. In contrast, in the case of the low-repulsion urethane foam 108, since the whole body contacts with the mattress uniformly, the small movement of the body based on heartbeats or breathing is likely transmitted to the mattress. As a result, since the small movement of the body is likely transmitted to the piezoelectric sensor, the displacement of the piezoelectric sensor due to the small movement of the body is made larger. When the low-repulsion urethane foam is used in this manner, the small movement of the body based on heartbeats or breathing can be detected with better sensitivity as compared with the case of using the usual urethane foam.

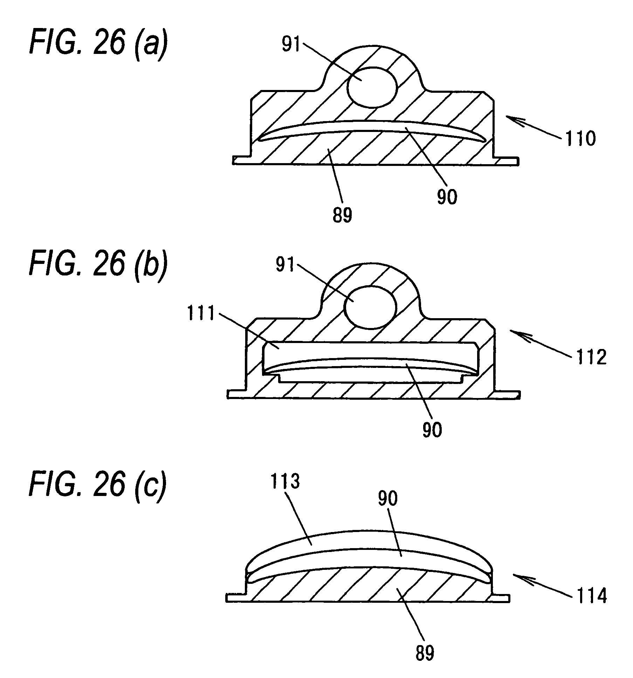

Next, an modified example of the second embodiment will be explained. This example shows another configuration of the pressure sensitive sensor. Fig. 26(a) is a sectional diagram of the pressure sensitive sensor which is arranged to space between a cable-shaped piezoelectric sensor and a non-linear flexible member. Fig. 26(b) is a sectional diagram of the pressure sensitive sensor which is arranged to be provided with a hollow portion. Fig. 26(c) is a sectional diagram of the pressure sensitive sensor which is arranged to use a flexible piezoelectric sensor of a sheet shape. The explanation will be made based on the drawings.

Fig. 26(a) shows the pressure sensitive sensor 110 which is arranged to space between the cable-shaped piezoelectric sensor 91 and the non-linear flexible member 90. When a bedded person 82 touches the pressure sensitive sensor 110 to apply a load thereto, firstly the cable-shaped piezoelectric sensor 91 deforms, then an elastic member between the cable-shaped piezoelectric sensor 91 and the non-linear flexible member 90 is sufficiently compressed and thereafter the non-linear flexible member 90 deforms. Thus, when a bedded person 82 starts to touch to the sensor and then the cable-shaped piezoelectric sensor 91 deforms, it is possible to determine the touch of the bedded person 82 before the deformation of the non-linear flexible member 90.

Fig. 25(b) shows a pressure sensitive sensor 112 which is configured by additionally providing the hollow portion 111 to the configuration of Fig. 25(a). According to this configuration, when a bedded person 82 touches to the sensor, since the cable-shaped piezoelectric sensor 91 is further likely to deform, the output signal becomes larger and so the determination of the contact can be made easily.

Fig, 26(c) is a sectional diagram of a pressure sensitive sensor 114 in which the flexible piezoelectric sensor of a sheet shape 113 is used as the piezoelectric sensor. In this case, since the sheet -shaped piezoelectric sensor 113 is used, the sensor is planar unlike the first embodiment and so the pressure sensitive sensor 114 is highly practical.

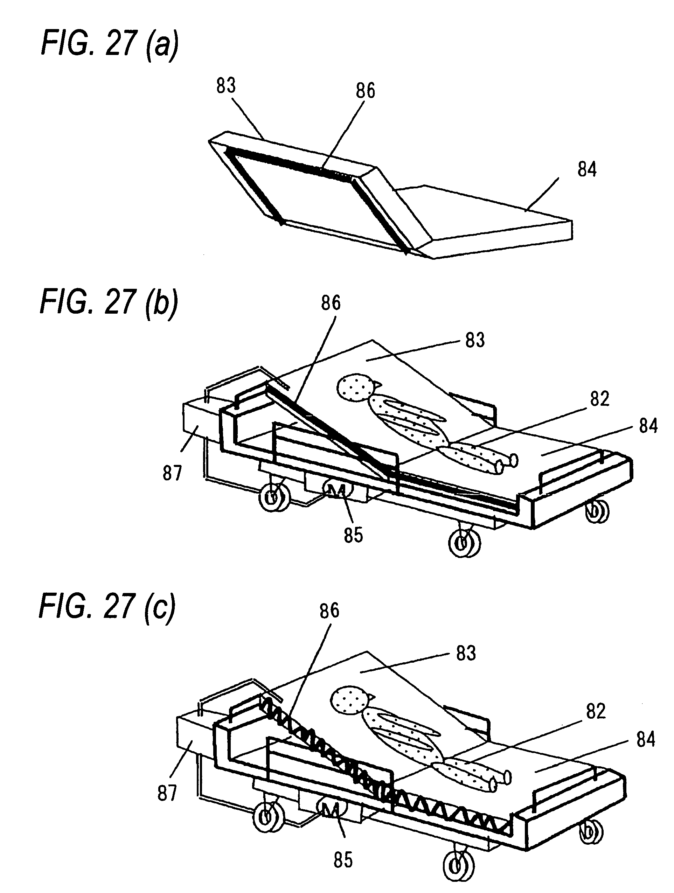

Incidentally, although the aforesaid embodiments are arranged in a manner that the pressure sensitive sensor 86 is disposed on the major surface of the back raising panel portion 83 and the knee raising panel portion 84, another configuration may be employed. For example, as shown in Fig. 27(a), the pressure sensitive sensor 86 is disposed on the rear surfaces of the back raising panel portion 83 and the knee raising panel portion 84, whereby when the contact of a subject on the rear surface of the back raising panel portion 83 and the knee raising panel portion 84 is detected at the time of the lowering driving of the back raising panel portion 83 and the knee raising panel portion 84, the lowering driving is stopped or the driving direction is reversed. Thus, at the time of the lowering driving, the caught between the back raising panel portion 83 or the knee raising panel portion 84 and the head board or foot board of the electrically driven bed can be prevented.

Further, as shown in Fig. 27(b), the pressure sensitive sensor 86 may be disposed at the side surfaces of the back raising panel portion 83 and the knee raising panel portion 84. In this case, a subject is prevented from being inadvertently caught between the side surface and the side fence 88.

Furthermore, the pressure sensitive sensor may be disposed so as to extend over the major surface and the rear surface of the back raising panel portion 83 and the knee raising panel portion 84. In this case, a subject is prevented from being inadvertently caught between the side fence 88 and all the surfaces including the major surface, the side surface and the rear surface of the back raising panel portion 83 and the knee raising panel portion 84.

In the case where the back raising panel portion 83 and the knee raising panel portion 84 are provided with a mattress, as shown in Fig. 27(c), the pressure sensitive sensor 86 may be disposed in a zigzag manner on the side surfaces of the mattress. In this case, the pressure sensitive sensor 86 is configured only by the piezoelectric sensor without containing any non-linear flexible member. Since the pressure sensitive sensor has many bent portions due to the zigzag shape, the curvature of the deformation portion becomes large when a subject directly or indirectly contacts with the pressure sensitive sensor and so the acceleration of the deformation also becomes large. Thus, the sensitivity of the pressure sensitive sensor is improved and so the contact with a subject can be detected easily. Further, since the pressure sensitive sensor is disposed on the side surfaces of the mattress, a bedded person does not feel a sense of incongruity in a laying state on the bed.

### Embodiment 3

Hereinafter, as a bed according to the third embodiment of the invention, a bed etc. capable of detecting heartbeats will be explained in detail with reference to drawings.

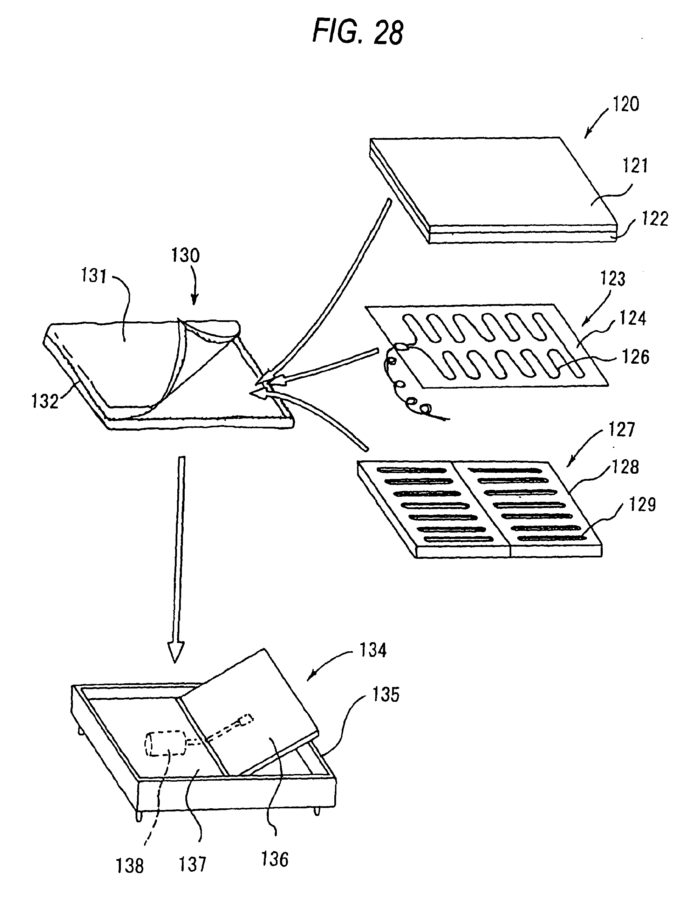

Fig. 28 is an exploded perspective view of the bed according to the third embodiment of the invention.

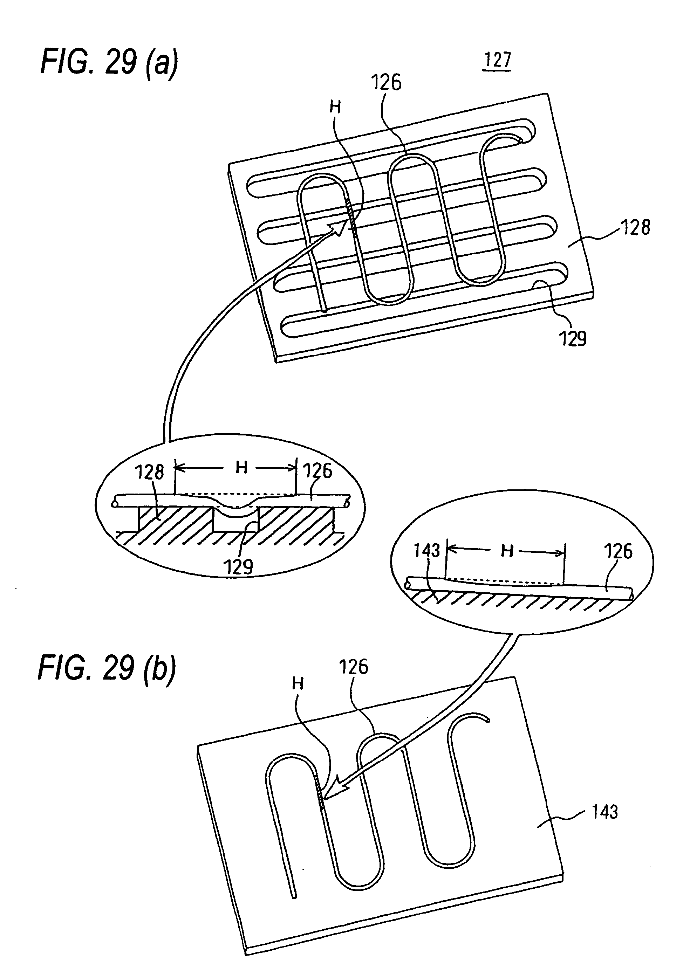

Fig. 29 is a perspective view showing the attachment state of a cord-shaped piezoelectric sensor of the apparatus.

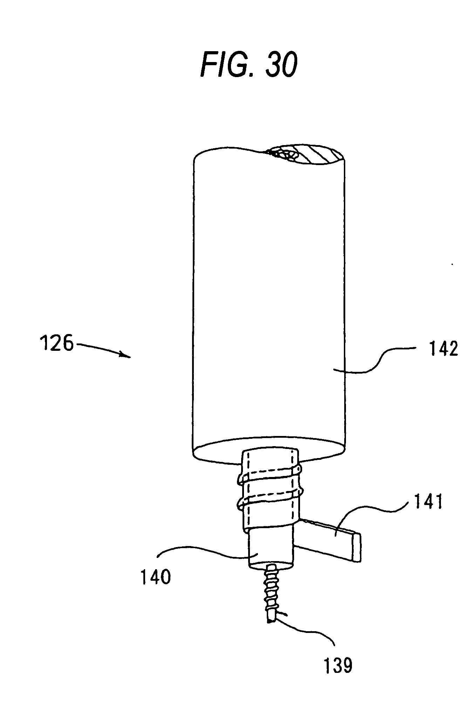

Fig. 30 is a configuration diagram of the cord-shaped piezoelectric sensor of the apparatus.

Firstly, the cord-shaped piezoelectric sensor used in this embodiment will be explained briefly.

The cord-shaped piezoelectric sensor is a cable-shaped sensor using piezo element material practically developed by the applicant of the invention. The configuration of the piezoelectric sensor is shown in Fig. 30.

In Fig. 30, 126 depicts a cord-shaped piezoelectric sensor, which is configured in a manner that piezo element material 140 is covered around the circumferential periphery of a center electrode 139 which is formed by a core wire (center electrode) 139 provided at the axial-direction center of the sensor, then an outer electrode 141 is disposed around the circumferential periphery of the piezo element material 140 and PVC (polyvinyl chloride resin) 142 is covered around the outermost periphery. Since this piezoelectric sensor is similar to that shown in Fig. 5, the detailed explanation thereof is omitted.

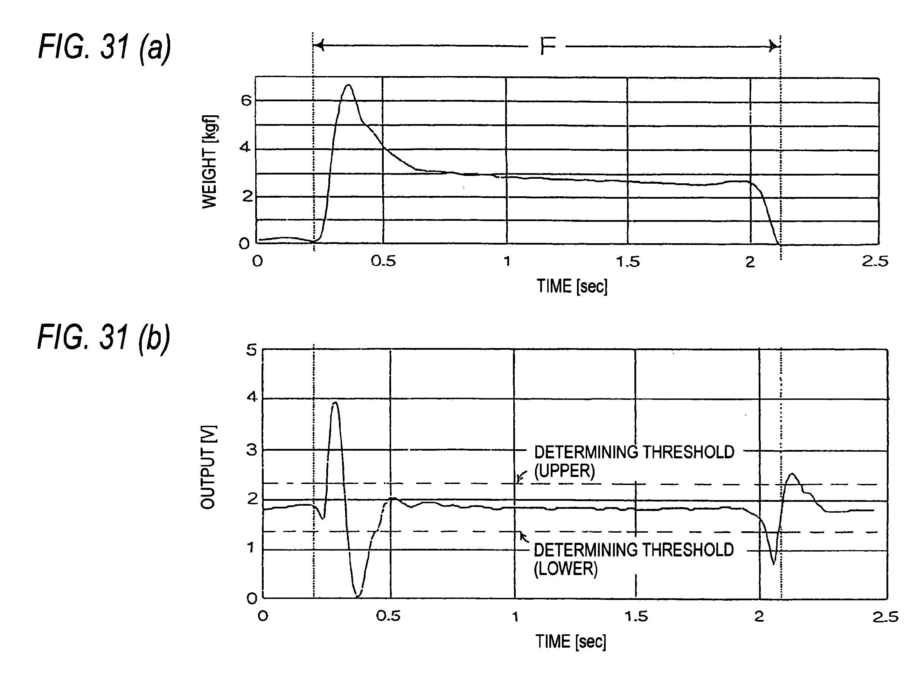

Fig. 31 is diagrams showing a load applied to the cord-shaped piezoelectric sensor 126 and sensor output characteristics thereof. The applicant experimented the relation between a load applied to the cord-shaped piezoelectric sensor 126 and the sensor output characteristics thereof, and found that the sensor output exhibited the phenomenon shown in (b) when a bending load shown in (a) was applied to the cord-shaped piezoelectric sensor 126.
(1) That is, at a time point t0 where no load was applied to the cord-shaped piezoelectric sensor 126, the sensor output was 2 (V).
(2) When a bending load was applied to the cord-shaped piezoelectric sensor 126 in a constant direction at a time point t1, the sensor output increased to 4 (V) instantaneously after the application of the load, then immediately reversed to 0 (V) and then returned to 2 (V) again.
(3) Thereafter, the sensor output was kept to 2 (V) even when the bent state was maintained.
(4) When the cord-shaped piezoelectric sensor 126 was restored to the original state at a time point t3, the sensor output reduced to 0.8 (V) instantaneously after the restoring, then immediately reversed to 2.2 (V) and then returned to 2 (V) again.

In this manner, since this cord-shaped piezoelectric sensor generates the output in response to the acceleration, this sensor outputs a signal only at the moment where a force applied thereto, and does not generates the output any more even if the force is continuously applied thereafter so long as the sensor does not deform, that is, there is no acceleration. Similarly, the sensor has characteristics that it also generates the output only at the moment where a force having been applied thereto is removed. Thus, even if this cord-shaped piezoelectric sensor is disposed at the lower portion of the bed in a wave-shaped manner, although the sensor is placed in an on state at the moment where it is bent but does not generate any output after completion of the displacement. Thereafter, the cord-shaped piezoelectric sensor generates the output only when a force is applied to a part thereof.

Fig. 35(a) is an experimental example further though of by the applicant of the invention so as to eliminate the drawbacks of the conventional apparatuses shown in (b) and (c).

In Fig. 35(a), 164 depicts a bed configured by a low-repulsion urethane layer, 126 a cord-shaped piezoelectric sensor disposed thereunder, and P a person laying on the bed.

According to this configuration, the vibration of heartbeats could be surely detected despite that the cord-shaped piezoelectric sensor 126 was disposed away from a person laying on the bed.

This reason is considered that since the low-repulsion urethane layer 121 is used as the bed, the portions of the back side of a person such as a neck, s shoulder, a back, a waist, a hip, thighs, calves, heels of legs etc. from a head side sink uniformly, sot that the vibration of heartbeats is transmitted from the whole surface of the urethane layer to the cord-shaped piezoelectric sensor as shown in the figure.

Fig. 28 is an exploded perspective view of the bed according to the third embodiment which is thought of based on the experimentation results of Fig. 35.

In the figure, 120 depicts a bed pad according to the invention, which is configured by the low-repulsion urethane layer 121 and a usual urethane layer 122 thereunder. The thickness of the low-repulsion urethane layer 121 is 1/2 or less as large as the entire thickness of the bed pad 120.

123 depicts a cord-shaped piezoelectric sensor laying sheet, which is configured by a cloth sheet 124 like a sheet and the cord-shaped piezoelectric sensor 126 which is disposed thereon in a wave-shaped manner.

127 depicts an uneven plastic plate which unevenness is formed by providing many recesses 129 on the surface of a plastic plate 128. The function and effects of the unevenness will be described later.

130 depicts a cover bag which is formed in a manner that a large sheet 131 is folded in two and three sides thereof are made so as to be opened and closed freely by a fastener 132. The fastener 132 is opened, then the bed pad 120, the cord-shaped piezoelectric sensor laying sheet 123 and the uneven plastic plate 127 are laminated in this order and housed within the cover bag 130 in the laminated state, and then the fastener 132 is closed, whereby a cushion portion of the bed is completed.

134 depicts a bed frame in which supporting plates 136, 137 are attached within a picture-frame shaped frame 135. The supporting plate (movable plate) 136 is arranged to incline by the operation of a motor 138 as shown by the figure. The cover bag 130 is placed on the supporting plates 136, 137 thereby to complete the bed.

The explanation will be made as to "the low-repulsion urethane" and "the usual urethane" constituting the bed pad 120.

The "low-repulsion urethane" is formed in a manner that the composition of urethane foam, that is, the kind of polyisocyanate, the number of functional group and the hydroxyl value of polyol are selected and constituted so as to cause the glassy transition at a temperature (usually a room temperature) where the urethane foam is used, thereby providing the low-repulsion property by the glassy transition phenomenon.

In contrast, the "usual urethane" is constituted so as not to cause the glassy transition at a temperature (a room temperature) where the urethane foam is used, thereby providing the high-repulsion property

The physical property values of the low-repulsion urethane and the usual urethane are as follows.

The usual urethane foam is classified into three kinds according to the hardness, that is, a soft type, a medium type and a hard type.
1) The urethane foam of the soft type has a density of 20 ± 2 kg/m³, a hardness of 6 ± 1.5, a tear strength of 0.2 kg/cm or more, a tensile strength of 0.6 kg/cm² or more, an elongation percentage of 150% or more, an impact resilience of 35% or more and a residual strain of 6% or less.
2) The urethane foam of the medium type has a density of 20 ± 2 kg/m³, a hardness of 11 ± 1.5, a tear strength of 0.2 kg/cm or more, a tensile strength of 0.7 kg/cm² or more, an elongation percentage of 120% or more, an impact resilience of 35% or more and a residual strain of 6% or less.
3) The urethane foam of the hard type has a density of 21 ± 2 kg/m³, a hardness of 15 ± 2.0, a tear strength of 0.2 kg/cm or more, a tensile strength of 0.7 kg/cm² or more, an elongation percentage of 120% or more, an impact resilience of 35% or more and a residual strain of 6% or less.

The low-repulsion urethane foam has a density of 65 ± 10 kg/m³, a hardness of 5.5 ± 2.0, a tear strength of 0.2 kg/cm or more, a tensile strength of 0.5 kg/cm² or more, an elongation percentage of 150% or more, an impact resilience of 5% or less and a residual strain of 3% or less.

The aforesaid densities and the hardnesses were measured by JIS-K6401, the tear strengths, the tensile strengths and the elongation percentage were measured by JIS-K6301, and the impact resiliences and the residual strains were measured by JIS-K6401.

Although the bed pad may be configured only by the low-repulsion urethane layer, the bed pad 120 is configured by the two-layer structure of the low-repulsion urethane layer 121 and the usual urethane layer 122 as shown in Fig. 28. This is because when the bed pad with a predetermined thickness is formed by only the low-repulsion urethane layer, the bed pad becomes unfavorably expensive and sinks too lowly.

In this case, it was proved to be preferable to use such a bed pad which was arranged in a manner that a half or less of the predetermined thickness was formed by the low-repulsion urethane layer, and an urethane layer of usual cushion material with the remaining thickness was adhered under the low-repulsion urethane layer.

Fig. 29 is schematic perspective views, in which, in order to improve the detection sensitivity of the cord-shaped piezoelectric sensor laying sheet 123, (a) shows a case where the cord-shaped piezoelectric sensor laying sheet 123 is disposed on an uneven plate and (b) shows a case where the cord-shaped piezoelectric sensor laying sheet is disposed on a flat plate.

In the case where the cord-shaped piezoelectric sensor 126 is provided on a flat plate 143 as shown in Fig. 29(b), if a portion H shown in a hatched manner of the cord-shaped piezoelectric sensor 126 is pushed down from the upper direction, since the pushed portion of the cord-shaped piezoelectric sensor is depressed downward from the state before the depression (a dotted line) as shown by an enlarged blowing diagram, so that only the displacement of the portion appears as a signal.

In contrast, in the case where the cord-shaped piezoelectric sensor 126 is provided on the uneven plastic plate 127 provided with the recesses 129 as shown in Fig. 29(a), if a portion H shown in a hatched manner of the cord-shaped piezoelectric sensor 126 is pushed down from the upper direction, the displacement in the elevational direction between before and after the depression of the cord-shaped piezoelectric sensor appears as a signal as shown by an enlarged blowing diagram. Further, since a large bending deformation appears at the edge of the recess 129 at the pushed portion of the cord-shaped piezoelectric sensor on the recess 129, a signal due to the bending deformation is also generated. The signal generated by the bending deformation is quite larger than the signal generated by the displacement in the elevational direction.

Thus, it is preferable to provide the uneven plastic plate 127 under the cord-shaped piezoelectric sensor 126. Incidentally, the bed pad may be configured as a bed pad of a sandwiched type in which the cushion lawyer made of usual urethane is disposed on the upper and lower surfaces of the low-repulsion urethane layer, and the piezoelectric sensor may be disposed on the lower surface of the bed pad of a sandwiched type.

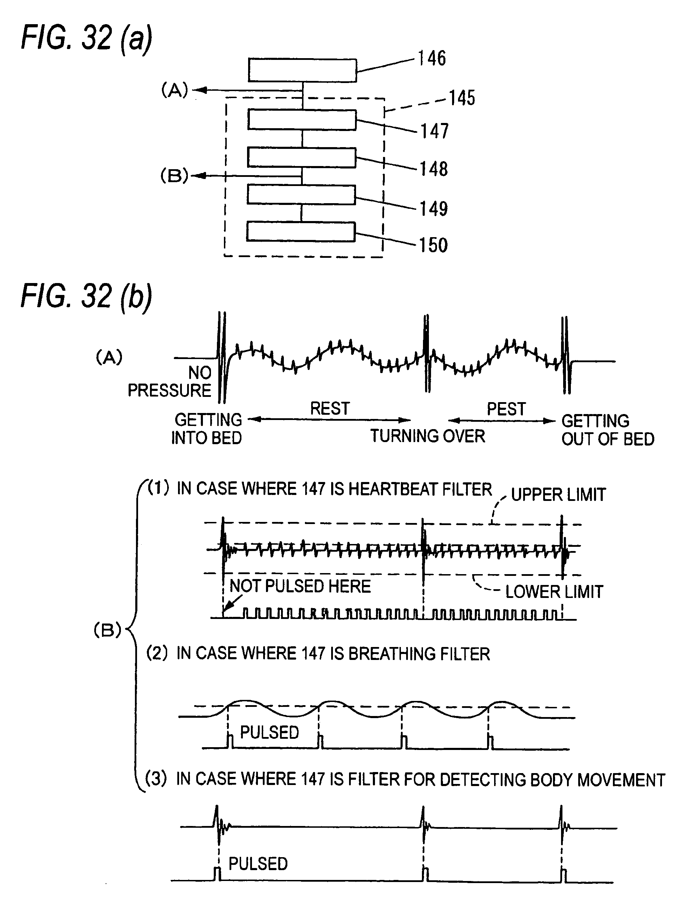

The heartbeats and breathing of a person on the bed pad can be detected through the known signal processing based on the output signal of the cord-shaped piezoelectric sensor according to the embodiment. Fig. 32 shows a block diagram (a) of a detection unit 145 for detecting heartbeats and breathing and a diagram (b) for explaining the theory for converting into pulse signals the detection signal from the cord-shaped piezoelectric sensor generated in response to heartbeats, breathing or the movement of the body caused by turning-over etc.

In Fig. 32(a), 145 depicts the detection unit for detecting heartbeats, breathing, turning-over etc., 146 the cord-shaped pressure sensitive sensor (cord-shaped piezoelectric sensor), 147 a filter circuit, 148 an amplifier circuit, 149 a counter circuit and 150 a display unit.

As shown in Fig. 32(a), when the cord-shaped pressure sensitive sensor (cord-shaped piezoelectric sensor) 146 deforms by heartbeats, breathing or the movement of the body caused turning-over etc. of a person on the bed, the cord-shaped pressure sensitive sensor 146 generates the voltage waveform as shown in (A) of the figure (b) due to the piezoelectric effect. That is, the amplitude of the detection voltage is large in the case of getting into the bed, turning over in the bed and getting out the bed, whilst the detection voltage caused by breathing and heart beats is quite small in its amplitude but periodical. The frequency of the detection voltage differs between the former case and the latter case. By notifying this feature, the filter circuit 147 passes, among the generated voltage outputs, only the predetermined frequency component corresponding to at least one of the heartbeat activity, the breathing activity and the body movements such as the turning-over, and the amplifier circuit 148 amplifies the predetermined frequency component thus passed. Further, the counter circuit 149 counts the number of times where the output signal of the amplifier circuit increases to a predetermined threshold value or more, whereby the number of the heartbeats, the number of the breathings and the number of turning-over are pulsed as shown in (1), (2) and (3) in the figure, respectively, and the number of the pulses is counted in each case and displayed on the display unit 150.

The band width of the filter circuit 147 is set in a range of about 0.7 to 2 Hz in the case of counting the number of heartbeats, set in a range of about 0.1 to 0.5 Hz in the case of counting the number of breathings and set in a range of about 1 to 10 Hz in the case of counting the number of the body movement such as turning-over. When the body is moved in the case of counting the number of heartbeats, since the level of the output signal caused by the body movement is larger than that caused by the heartbeats, the output signal is distorted and so an erroneous operation may be caused. Thus, an upper limit and a lower limit are provided in the case of counting the number of heartbeats, and the number of times corresponding to the heartbeats where the output signal exceeds the predetermined threshold value is counted in the case where the output signal exists within the range between the upper and lower limits. The similar procedure is performed in the case of counting each of the number of breathings and the number of the body movement.

In the aforesaid example, although each of the number of heartbeats and the number of breathings is counted by counting the number of times where the output signal increases to the predetermined threshold value or more, another known method may be employed. For example, a method may be employed in which the output signal of the pressure sensitive sensor 146 is converted into a digital data through the A/D conversion by a microcomputer, and an autocorrelation coefficient is calculated from the time-series data of the digital data thereby to calculate the number of heartbeats and the number of breathings.

Further, although the cord-shaped pressure sensitive sensor is used as the cord-shaped pressure sensitive sensor, another sensor capable of detecting vibration such as an electrostatic capacitance sensor of a coaxial shape, a pressure sensitive resistance type sensor of a cable shape may be used instead thereof. Further, a belt-shaped or sheet-shaped pressure sensitive sensor as well as a cable-shaped one may be used so long as it is possible to be disposed on the lower surface of the bed pad and capable of detecting the vibration due to heartbeats or breathings of a human body.

### Industrial Applicability

According to the bed and the in-bed state detection method of the invention, the first detection means and the second detection means each formed by the cable-shaped pressure sensitive sensor are disposed along the laying direction at the both end sides of the bedding surface, then the determination means compares the output signals from the first detection means and the second detection means thereby to determine the positional shift of a bedded person on the bedding surface toward the side portion, thereby preventing the bedded person from falling from the bedding surface in advance in accordance with the determination result.

Further, since the cable-shaped pressure sensitive sensors are merely disposed at the both end sides of the bedding surface, the cost-up can be suppressed as much as possible and also the degradation of the comfortability to sleep can be suppressed as much as possible as compared with a case where sensors each formed by an expensive piezoelectric elements are disposed on the bedding surface in a lattice pattern.

The invention is arranged to control the pressure sensitive sensor disposed at the entire periphery or the part of the surface end portion of at least one of the back raising panel portion and the knee raising panel portion, the determination means for determining the touch of a bedded person to the pressure sensitive sensor based on the output signal from the pressure sensitive sensor, and the driving means for elevationally driving the back raising panel portion and the knee raising panel portion in accordance with the determination signal from the determination means. Thus, when it is determined that a bedded person contacts with the pressure sensitive sensor during the elevational operation of the back raising panel portion or the knee raising panel portion, such control can be performed that the elevational driving of the back raising panel portion or the knee raising panel portion is stopped or the driving direction is reversed. As a result, an inadvertent caught at the time of the elevational movement of the bed can be prevented.

Further, the bed pad is configured by the low-repulsion urethane layer and the piezoelectric sensor disposed on the lower surface of the low-repulsion urethane layer or configured by the low-repulsion urethane layer, the cushion lawyer made of usual urethane disposed on the lower surface thereof and the piezoelectric sensor disposed on the lower surface of the cushion layer, so that such a bed pad can be provided which can surely detect heartbeat vibration and is good in the comfortability of the bed.

Furthermore, when a rigid plate having unevenness formed on the surface thereof is added, the heartbeat vibration can be detected more surely. When the bed is formed by using such the bed pad, the bed comfortable to sleep can be obtained.

## Claims

1. A bed for detecting shift of a position of a bedded person on a bedding surface, comprising:
a first pressure sensitive sensor and a second pressure sensitive sensor each having a cable shape, disposed separately along side edges of the bedding surface at both end sides in a width direction of the bedding surface, respectively, each of which detects an acceleration component caused by a movement of a bedded person; and
determination means which compares output signals from the first pressure sensitive sensor and the second pressure sensitive sensor to determine a shift state of the bedded person toward a side potion of the bedding surface.

2. A bed according to claim 1, wherein the first pressure sensitive sensor and the second pressure sensitive sensor are arranged in a manner that a wiring density on a head portion side of the bedded person is set to be higher than other region.

3. A bed according to claim 1 or 2, wherein the first pressure sensitive sensor and the second pressure sensitive sensor are arranged in a manner that a wiring density on a leg portion side of the bedded person is set to be lower than other region.

4. A bed according to claim 2 or 3, wherein the first pressure sensitive sensor and the second pressure sensitive sensor are wired in a wave shape on the bedding surface, and the wiring density is changed in accordance with an interval between adjacent waves of the wave shape.

5. A bed according to one of claims 1 to 4, wherein projection pieces are disposed on an upper side or a lower side of the pressure sensitive sensor and are overlapped on the pressure sensitive sensor at plural portions thereof.

6. A bed according to claim 5, wherein the pressure sensitive sensor is adhered to a sheet and the projection pieces are adhered to a sheet, and the pressure sensitive sheet to which the pressure sensitive sensor is adhered and the projection piece sheet to which the projection pieces are adhered are laminated to each other.

7. A bed according to one of claims 1 to 6, wherein the determination means outputs a notification signal when it is determined that positional shift of the bedded person on the bedding surface arises, and notification means is further provided which notifies occurrence of shift in response to the notification signal outputted from the determination means.

8. An in-bed detection method of a bed for detecting shift of a position of a bedded person on a bedding surface, comprising the steps of:
obtaining a ratio between intensities of output signals from a first pressure sensitive sensor and a second pressure sensitive sensor each having a cable shape, disposed separately along side edges of the bedding surface at both end sides in a width direction of the bedding surface, respectively, each of which detects an acceleration component caused by a movement of a bedded person; and
determining that positional shift of the bedded person on the bedding surface arises when the ratio is in a predetermined range set in advance.

9. In a bed which includes at least one of a back raising panel portion and a knee raising panel portion and driving means which performs elevational driving of at least one of the back raising panel portion and the knee raising panel portion, and which is capable of attaching a side fence, the bed is **characterized by** comprising:
a pressure sensitive sensor which is disposed at an entire periphery or a part of a surface end portion of at least one of the back raising panel portion and the knee raising panel portion;
determination means which determines touch of a bedded person to the pressure sensitive sensor based on an output signal from the pressure sensitive sensor; and
control means which controls the driving means in accordance with a determination signal from the determination means.

10. A bed according to claim 9, wherein the pressure sensitive sensor is formed by a non-linear flexible member which displacement amount with respect to a load is non-linear and a piezoelectric sensor with flexibility which deforms according to a displacement of the non-linear flexible member.

11. A bed according to claim 10, wherein the non-linear flexible member is configured by a thin type elastic material which is formed in a belt shape and has a convex portion.

12. A bed according to claim 10 or 11, wherein the non-linear flexible member and the piezoelectric sensor are disposed in deformation means capable of deforming according to a load.

13. A bed according to claim 12, wherein the deformation means has a hollow portion so that at least one of the non-linear flexible member and the piezoelectric sensor can be deformed easily.

14. A bed according to one of claims 9 to 13, wherein that the determination means determines whether or not a bedded person continues to touch the pressure sensitive sensor based on an output signal from the pressure sensitive sensor.

15. A bed according to one of claims 9 to 14, wherein the control means is arranged to validate the determination signal from the determination means when the driving means performs the elevational driving and to invalidate the determination signal from the determination means when the driving means does not perform the elevational driving.

16. A bed comprising:
a low-repulsion urethane layer; and
a pressure sensitive sensor disposed on a lower surface of the low-repulsion urethane layer.

17. A bed comprising:
a low-repulsion urethane layer;
a cushion lawyer made of usual urethane disposed on a lower surface of the low-repulsion urethane layer; and
a pressure sensitive sensor disposed on a lower surface of the cushion lawyer.

18. A bed according to claim 16 or 17, wherein the pressure sensitive sensor is configured by a cord-shaped piezoelectric sensor with flexibility.

19. A bed comprising:
a rigid plate having unevenness formed on a surface thereof; and
a bed pad which is disposed on the rigid plate and recited in one of claims 16 to 18.

20. A bed comprising:
a bed frame;
a movable plate attached on the bed frame so as to be able to incline; and
a bed element which is disposed on the movable plate and recited in claim 19.
